# EUROPEAN PATENT APPLICATION

(11) **EP 2 399 935 A2**
(43) Date of publication of application: **28.12.2011**
(21) Application number: 11173253.3
(22) Date of filing: 15.02.2005
(51) Int. Cl.: C07K 16/28, A61K 39/395, C12P 21/00

(54) **An anti-CD137 antibody as an agent in the treatment of cancer and glycosylation variants thereof**

(62) Divisional of application: 05723079.9
(71) Applicant: GTC Biotherapeutics, Inc., Framingham, MA 01702 (US)
(72) Inventor: Strome, Scott, Rochester, MN Minnesota 55902 (US); Schindler, Daniel, Newton Upper Falls, MA Massachusetts 02464 (US); Chen, Lieping, Sparks Glencoe, MD Maryland 21152 (US); Meade, Harry, Newton, MA Massachusetts 02158 (US)
(74) Representative: Williams, Gareth Owen

(57) **Abstract**

The current invention relates to the development and methods of use of a recombinant agonistic antibody anti-human CD 137, and glycosylation variants thereof. These antibodies act as an anti-cancer agents and/or immune modulators that are effective in shrinking solid tumors or other cancerous indications and preventing their recurrence. The types of cancer for which the contemplated antibody is effective in treating also include leukemia and lymphoma. In a preferred embodiment the recombinant antibodies of the current invention were produced in and purified from the milk of transgenic animals.

## Description

### FIELD OF THE INVENTION

The present invention relates to the recombinant production of an agonistic anti-CD137 antibody and variants thereof. In particular, the current invention provides for the transgenic production of anti-CD137 antibodies, in which the glycosylation profiles of the antibodies are altered to enhance their use in the treatment of specific types of cancers or other disease states.

### BACKGROUND OF THE INVENTION

As stated above, the present invention relates generally to the field of the recombinant production of therapeutic antibodies and the modification of their glycosylation profile. More particularly, it concerns improved methods for generating transgenic agonistic anti-CD137 antibodies optimized for the treatment of various types of cancer and/or autoimmune disorders.

Glycosylation is involved in the correct folding, targeting, bioactivity and clearance of therapeutic glycoproteins. With the development of transgenic animals as expression systems it is important to understand the impact of different genetic backgrounds and expression levels on glycosylation. As will be seen the glycosylation profile of recombinant anti-CD137 antibody and other proteins of interest produced in several transgenic goat lines, from cloned animals and at different stages of lactation including induced lactations was evaluated.

Recently, systematic efforts have been undertaken to produce and characterize proteins with defined alterations in carbohydrate structure. Because it is becoming increasingly commonplace to express recombinant proteins in heterologous host cells, it is fundamentally important whether changes in carbohydrate structure due to species-specific glycosylation or cell growth conditions affect function, pharmokinetics and/or immunogenicity. Several approaches have been attempted to alter the glycosylation state of IgG antibodies: inhibition of glycosylation by culturing cells in the presence of the drug tunicamycin (Leatherbarrow et al. 1985; Walker et al. 1989; Pound et al. 1993); treatment of glycoproteins with specific glycosidases that remove the entire oligosaccharide or specific residues (Tsuchiya et al. 1989; Boyd et al. 1995); or site-directed mutagenesis to remove either the carbohydrate addition site (Tao, Smith et al. 1993) or residues within the CH2 region that contact the core oligosaccharide residues (Lund, Takahashi et al. 1996). These studies have confirmed that the presence of carbohydrate is essential to antibody function. However, site-directed mutagenesis alters the sequence of the protein, while glycosidase treatment is not completely efficient and the reaction conditions may adversely affect the resultant antibody or increase immunogenicity. These are variables that are difficult to overcome and may obscure interpretation of results.

Recombinant proteins provide effective therapies for many life-threatening diseases. The use of high expression level systems such as bacterial, yeast and insect cells for production of therapeutic protein is limited to small proteins without extensive post-translational modifications. Mammalian cell systems, while producing many of the needed post-translational modifications, are more expensive due to the complex, and therefore sophisticated culture systems are required. Moreover, in these sophisticated cell culture methods reduced protein expression levels are often seen. Some of the limitations of mammalian cell culture systems have been overcome with the expression of recombinant proteins in transgenic mammals (Meade H 1998) or avians. Proteins have been produced in mammary glands of various transgenic animals with expression levels suitable for cost effective production at the scale of hundreds of kilograms of protein per year. Although the post-translational modification of proteins produced using transgenic technology has been published (Edmunds T 1998; James DC 1995), the effect of expression level and genetic polymorphisms on these post-translational modifications, especially glycosylation, has not been reported and is highly variable.

CD137 (also called 4-1BB) is a membrane glycoprotein that is inducibly expressed on activated T cells, B cells, dendritic cells and natural killer (NK) cells. Anti-human CD137 antibodies are potential biotherapeutic agents to shrink solid tumors *in vivo* and prevent their recurrence. CD137 is a member of the tumor necrosis factor receptor (TNFR) superfamily of costimulatory molecules. This molecule is inducibly expressed on activated T-, B-, dendritic and natural killer (NK) cells. Stimulation of CD137 by its natural ligand, CD137L, or by agonistic antibody induces vigorous T-cell proliferation and prevents activation-induced cell death. The intracellular biochemical pathway for CD137 signaling is not fully understood, but TNFR associated factors (TRAF) 1 and 2 are believed to play a role. The extensive effects of CD137 ligation on T-cell co-stimulation and survival and on dendritic and NK cell activation suggest that the CD 13 7 pathway plays a role in both innate and adaptive immune responses against cancers.

The development of therapeutic anti-CD137 would fill a critical unmet need for an effective immunomodulatory treatment of solid tumors. Despite significant advances in cancer therapy in recent decades, the majority of solid tumors in advanced stages have remained remarkably resistant to effective treatment. These include melanoma and carcinomas of the breast, colon, ovaries, kidney, prostate and lung. Agonistic anti-CD137 antibody has induced complete or partial regression in murine tumor models with diverse histological origin, either alone or in combination with other modalities. The development of a novel immuno-modulatory therapy would substantially reduce suffering and improve the quality of life for patients with these types of cancers. Moreover, and according to the current invention, an anti-CD137 also appears to ameliorate experimental autoimmune encephalo-myelitis and systemic lupus erythematosis in mouse models.

Accordingly, a need exists to genetically engineer forms of anti-CD137 antibodies in sufficient quantities for characterization and development as a potential anti-cancer and immunotherapeutic agent. A need likewise exists to better tailor the glycosylation profile of recombinantly produced antibodies for desired or diversed therapeutic effects.

### SUMMARY OF THE INVENTION

Briefly stated, according to the current invention there are two desirable types of recombinant CD137 antibody preparations that are optimized for use as human biotherapeutics: 1) a first preferred embodiment would entail constructing a fully glycosylated and humanized antibody containing, which should reduce or prevent inactivation of the therapeutic protein by Human Anti-Mouse Antibody (HAMA) response, while retaining activity against solid tumors and usefulness in conjunction in bone marrow transplant operations ("BMT"); and 2) a second preferred embodiment would entail constructing an aglycosylated form of an agonistic anti CD 137 antibody, which would offer simpler manufacture and separate indications of specific utility such as leukemia and lymphoma, as well as utility against autoimmune disease states.

Other objects of the current invention include the production of a humanized version of the agonistic antibody anti-human CD137, an immune modulator that is effective in shrinking solid tumors and preventing their recurrence.

Specific indications against which the antibody variants of the current invention would provide beneficial therapeutic effects include: an effective immunomodulatory treatment of solid tumors; melanomas; as well as carcinomas of the breast, colon, ovaries, kidney, prostate and lung.

In another embodiment of the current invention the anti-CD 137 antibodies of the invention are effective in the treatment of autoimmune derived encephalo-myelitis and systemic lupus erythematosis.

It should be noted that the preferred embodiments of the current invention include pharmaceutical compositions which comprise an amount of a transgenic protein of interest, a prodrug thereof, or a pharmaceutically acceptable salt of said compound or of said prodrug and a pharmaceutically acceptable vehicle, diluent or carrier.

This invention is also directed to pharmaceutical compositions for the treatment of disease conditions which may be optimally treated with biologically active protein molecules that have had their glycosylation profile changed or modified. These and other objects which were more readily apparent upon reading the following disclosure may be achieved by the present invention.

### BRIEF DESCRIPTION OF THE DRAWING

FIG. 1 Shows a graph of the spleen size of sacrificed mice after treatment with the antibodies of the current invention.

FIG. 2 Shows a graph of the survival time of animals treated with antibodies of the current invention according to different treatment regimens.

FIG. 3. Shows the antibodies of that invention biotinylated. Goat anti-human H&L-AP detect bound to both biotinylated and non-biotinylated antibody. Strep-AP only bound to the biotinylated antibody. Both glycosylated and glycosylated were bound equally.

FIG. 4 Shows a graph of the survival time of animals treated with antibodies of the current invention according to different treatment regimens.

FIG. 5 Shows Anti-human CD137 mAb binds to CHO/CD137 cells (left) and to CD3-activated human T cells (right). Flow cytometric analysis of GW, a mouse anti-human CD 137 mAb (bottom) is compared to binding by commercial anti-human CD137 and anti-human CD3 (top). FITC-labeled antibodies were used for either direct (anti-CD3) or indirect immunofluorescence. X axis, fluorescence intensity; Y axis, no. of cells. Histogram at left in each panel is isotype-matched control IgG.

FIG. 6 Shows the Co-stimulation of human T cell growth by anti-CD3 and anti-CD 137 mAb of the invention. T cell proliferation was measured by incorporation of radioactive thymidine.

FIG. 7 Shows a silver-stained SDS-PAGE gel of transgenic goat milk samples at different stages of antibody purification. Lane 1: milk sample containing human IgG1. Lane 2: Protein A eluate. Lane 3: CM HyperD column eluate; Lane 4: Methyl HyperD column eluate.

FIG. 8 Shows a histological representation of the tissues after various treatment regimens according to the antibodies of the invention.

FIG. 9 Shows flow cytometry data related to the bioactivity of antibodies produced by transgenic animals versus CHO cell produced 4-1BB at various doses.

FIG. 10 Shows flow cytometry data related to the bioactivity of antibodies produced by transgenic animals, glycosylated versus non-glycosylated.

FIG. 11 Shows flow cytometry data related to the bioactivity of antibodies produced by transgenic animals as measured by various bioactivity markers.

FIG. 12 Shows the activity of the antibodies of the invention in treatment regimen with 100 IU of IL-2. NK cells were separated from fresh Buffy Coat blood via Ficoll-Paque separation followed by positive selection CD56 PE and anti-PE magnetic beads. NK cells were cultured for four days in 100 IU/ml of IL-2 before being transferred to another plate coated with 10ug/ml of the appropriate protein. 24 hours later the supernatants were harvested for IFN gamma ELISA and the cells were triple stained with CD3 PerCP, CD56 PE, and CD137 FITC. The NK cells were analyzed by flow cytometry

FIG. 13 Shows the activity of the antibodies of the invention in treatment regimen with 200 IU of IL-2. NK cells were separated from fresh Buffy Coat blood via Ficoll-Paque separation followed by positive selection CD56 PE and anti-PE magnetic beads. NK cells were cultured for four days in 200 IU/ml of IL-2 before being transferred to another plate coated with 10ug/ml of the appropriate protein. 24 hours later the supernatants were harvested for IFN gamma ELISA and the cells were triple stained with CD3 PerCP, CD56 PE, and CD137 FITC. The NK cells were analyzed by flow cytometry.

FIG. 14 Shows a graph of 4-1BB activity versus an IGgl.

FIG. 15 Shows a general schematic describing the general production of transgenic mammals.

FIG. 16 Shows a graph of spleen size after treatment with the antibodies of the invention.

FIG. 17 Shows a graph of spleen size in whole animal models after time being treated with the antibodies of the invention.

FIG. 18 Shows activity of the antibodies of the invention in a cellular assay over various times after stimulation.

FIG. 19 Shows a graph of treatment with the antibodies of the current invention in the presence of IL-2 and/or γ interferon.

FIG. 20 Shows a general schematic of transgene constructs for milk expression of antibodies. The gene of interest replaces the coding region of caprine beta-casein, a milk specific gene. The 6.2 kb promoter region is linked to the coding regions of either the H or L IgG chains, followed by untranslated caprine beta casein 3' sequences and downstream elements. Black boxes: H and L exons; striped boxed: genomic introns; arrows: direction of transcription.

Fig. 21. Shows a comparison of the carbohydrates in anti CD137 antibodies from transgenic animal and human 293 cell line. The antibodies including both glycosylated and non-glycosylated forms from transgenic animals were expressed and purified, while the same antibody from human 293 cell line was expressed and purified. The antibodies in 5 ug were applied to a 4-20% SDS-PAGE in reducing condition and stained with Coomassie blue.

Fig. 22. Shows a comparison of the carbohydrates in anti CD137 antibodies from transgenic animal and human 293 cell line when applied to a 4-20% SDS-PAGE and transferred to a PVDF membrane. A western blot was performed using a goat anti human IgG (Fc specific) antibody.

Fig. 23(a) - (c) Shows a MALDI-TOF analysis of the carbohydrates. The carbohydrates were released using PNGase F in the presence of 1% β-mercaptoethanol from glycosylated antibodies.

Fig. 24(a)-(b) Shows chromatographs of glycosylated and non-glycosylated transgenic antibodies on Con A column.

Fig. 25(a)-(b) Shows the use of a Lentil lectin column used to determine the presence of core fucose, Both glycosylated and non-glycosylated transgenic antibodies were applied to a Lentil lectin column, respectively. The bound protein was eluted by α-methylmannoside.

FIG. 26. Shows response curves differences of the Antibodies of the invention over time versus controls.

FIG. 27. Shows a graph of NK cell ELISA for γ interferon. NK cells were separated from fresh Buffy Coat blood via Ficoll-Paque separation followed by positive selection CD56 PE and anti-PE magnetic beads. NK cells were cultured for four days in 100 IU/ml of IL-2 before being transferred to another plate coated with 10ug/ml of the appropriate protein. 24 hours later the supernatants were harvested for IFN gamma ELISA and the cells were triple stained with CD3 PerCP, CD56 PE, and CD137 FITC. The NK cells were analyzed by FACS.

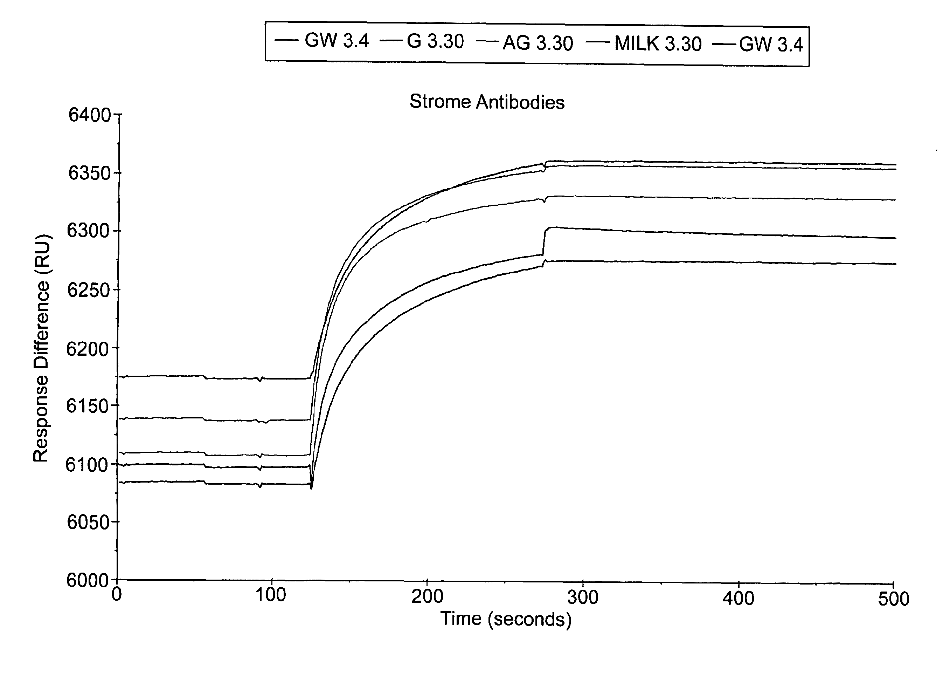

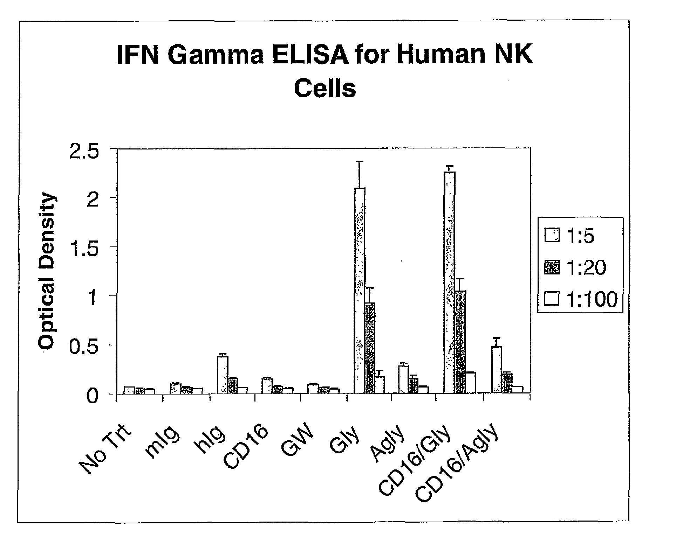

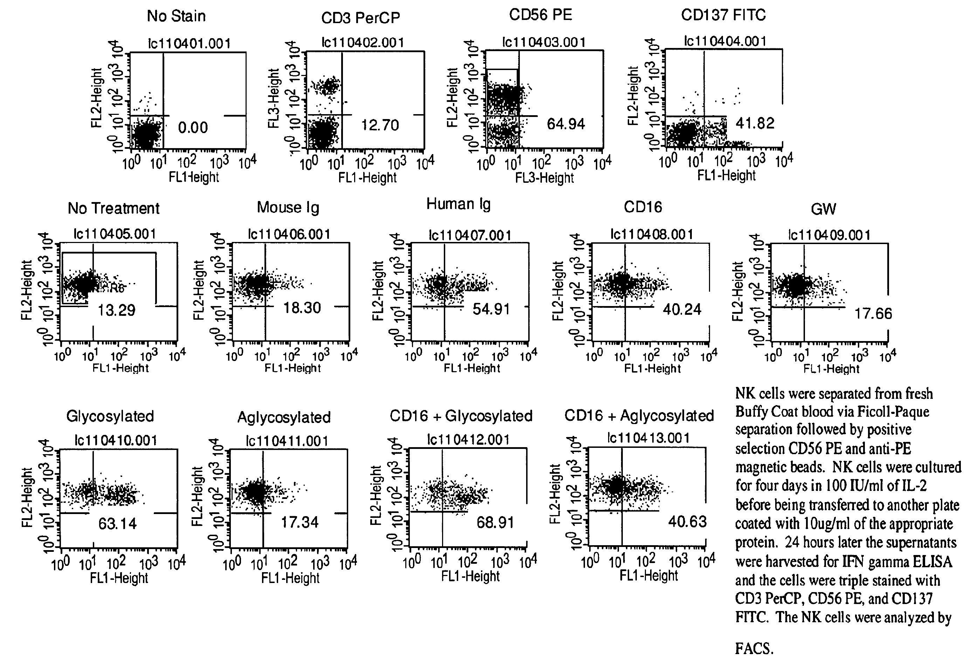

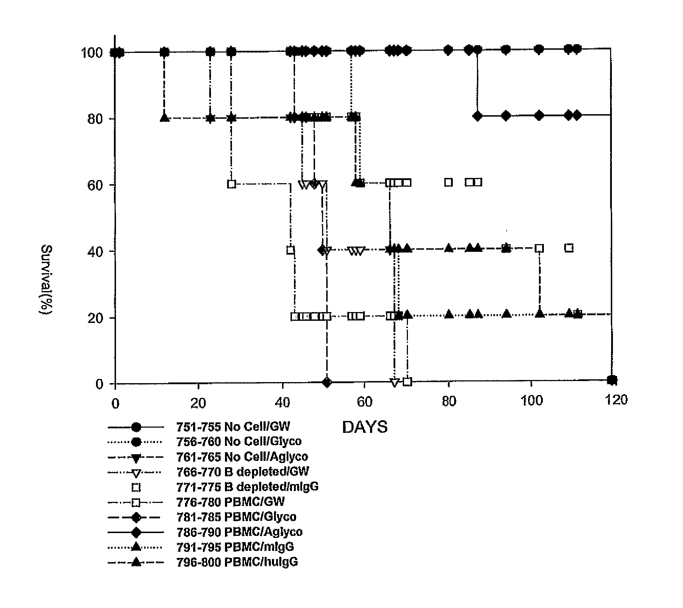

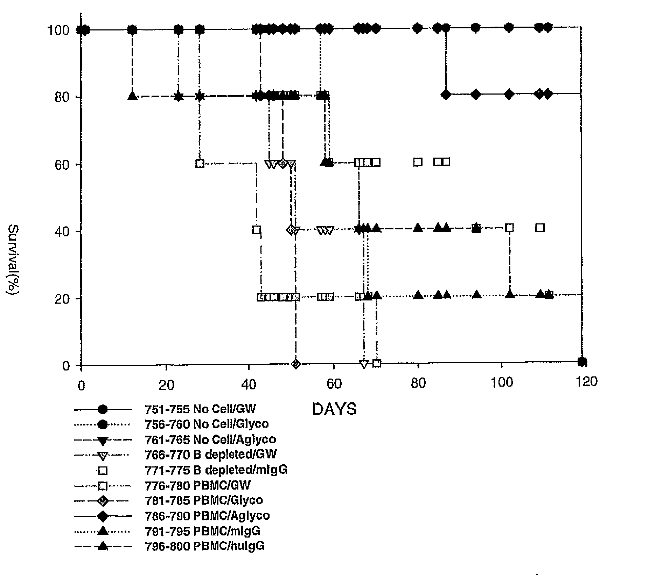

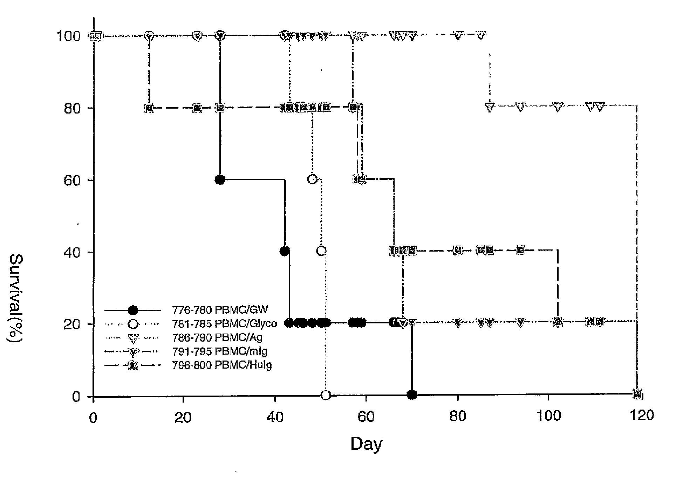

FIG. 28. Shows the activity of the antibodies of the invention in treatment regimen with 100 IU of IL-2. NK cells were separated from fresh Buffy Coat blood via Ficoll-Paque separation followed by positive selection CD56 PE and anti-PE magnetic beads. NK cells were cultured for four days in 100 IU/ml of IL-2 before being transferred to another plate coated with 10ug/ml of the appropriate protein. 24 hours later the supernatants were harvested for IFN gamma ELISA and the cells were triple stained with CD3 PerCP, CD56 PE, and CD137 FITC. The NK cells were analyzed by The NK cells were analyzed by flow cytometry.

FIG. 29. Shows a western blot of anti-CD137 production levels in the milk of various lines of transgenic mice.

FIG. 30. Shows a graph of the survival time of animals treated with antibodies of the current invention according to different treatment regimens.

FIG. 31. Shows a graph of the survival time of animals treated with antibodies of the current invention according to different treatment regimens with regard to PBMC.

FIG. 32. Shows a graph of the survival statistics of animals treated with antibodies of the current invention.

FIG. 33. Shows a figure of the survival statistics of animals in graphic form.

FIG. 34. Shows an ELISA assay of γ-interferon production from cell cultures exposed to antibodies of the invention. The ELISA measures supernatant γ-interferon, which is selectively stimulated by anti-CD137.

FIG. 35. Shows a bar graph of average spleen size.

FIG. 36. Shows a bar graph of mice with or without lymphoma.

FIG. 37. Shows the spleen sizes of the animals treated with the antibody variants of the invention. It appears that mice given PBMC and GW or glycosylated antibody die with massive splenomegaly. The B cell depleted animals treated with GW also die. Animals with antibody and no cells seem appear to be in good health. Likewise, animals with aglycosylated antibody and cells seem in good health.

### DETAILED DESCRIPTION

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well known in the art. Generally, nomenclatures used in connection with, and techniques of biochemistry, enzymology, molecular and cellular biology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. All publications, patents and other references mentioned herein are incorporated by reference.

In a preferred embodiment of the current invention the aglycosylated antibody is used in an immunotherapy against cancer and the development of cancerous tumors. The physiological pathway involved works through 4-1BB, stimulating its activity, to prolong survival.

The following abbreviations have designated meanings in the specification and are provided for convenience:

### Abbreviation Key:

BMT,
HPAEC, high.-pH anion-exchange chromatography;
PAD, pulsed amperometric detection;
HPLC, high-performance liquid chromatography;
MS, mass spectrometry;
MALDI, matrix-assisted laser desorption/ionization;
TOF, time of flight;
SDS-PAGE, sodium dodecyl sulfate-polyacrylamide gel electrophoresis;
FACE, Fluorophore-assisted carbohydrate electrophoresis;
AT, anti-CD137 antibody;
agonistic CD 137 antibody, recombinant human anti-CD137 antibody;
PNGase F, peptide N-glycosidase F;
Endo H, endo-β-N-acetylglucosaminidase H;
EHS, Engelbreth-holm-swarm;
CHO cells, Chinese hamster ovary cells;
sDHB, 2,5-dihydroxybenzoic acid matrix;
NeuAc, N-acetylneuraminic acid;
NeuGc, N-glycolylneuxaminic acid;
NT, Nuclear Transfer;
GlcNAc, N-acetylglucosamine;
GalNAc, N-acetylgalactosamine;
Gal, galactose; Man, mannose.

### Explanation of Terms:

Bovine - Of or relating to various species of cows.
Caprine - Of or relating to various species of goats.
Chimeric Antibody - A genetically engineered fusion of parts of a mouse antibody with parts of a human antibody. Generally, chimeric antibodies contain approximately 33% mouse protein and 67% human protein. Developed to reduce the HAMA response elicited by murine antibodies, they combine the specificity of the murine antibody with the efficient human immune system interaction of a human antibody.
Expression Vector - A genetically engineered plasmid or virus, derived from, for example, a bacteriophage, adenovirus, retrovirus, poxvirus, herpes virus, or artificial chromosome, that is used to transfer an biologically active transgenic protein coding sequence, operably linked to a promoter, into a host cell, such that the encoded recombinant transgenic protein is expressed within the host cell.
"Fully Human" Antibody - Recently the term "fully human" and "human" antibody has been used to label those antibodies derived from transgenic mice carrying human antibody genes or from human cells. To the human immune system, however, the difference between "fully human", "humanized", and "chimeric" antibodies may be negligible or nonexistent and as such all three may be of equal efficacy and safety.
Functional Proteins - Proteins which have a biological or other activity or use, similar to that seen when produced endogenously.
Homologous Sequences - refers to genetic sequences that, when compared, exhibit similarity. The standards for homology in nucleic acids are either measures for homology generally used in the art or hybridization conditions. Substantial homology in the nucleic acid context means either that the segments, or their complementary strands, when compared, are identical when optimally aligned, with appropriate nucleotide insertions or deletions, in at least about 60% of the residues, usually at least about 70%, more usually at least about 80%, preferably at least about 90%, and more preferably at least about 95 to 98% of the nucleotides. Alternatively, substantial homology exists when the segments did hybridize under selective hybridization conditions, to a strand, or its complement. Selectivity of hybridization exists when hybridization occurs which is more selective than total lack of specificity. Typically, selective hybridization did occur when there is at least about 55% homology over a stretch of at least about 14 nucleotides, preferably at least about 65%, more preferably at least about 75%, and most preferably at least about 90%.
Humanized Antibody -A genetically engineered antibody in which the minimum mouse part from a murine antibody is transplanted onto a human antibody; generally humanized antibodies are 5-10% mouse and 90-95% human. Humanized antibodies were developed to counter the HAMA and HACA responses seen with murine and chimeric antibodies. Data from marketed humanized antibodies and those in clinical trials show that humanized antibodies exhibit minimal or no response of the human immune system against them.
Leader sequence or a "signal sequence," - a nucleic acid sequence that encodes a protein secretory signal, and, when operably linked to a downstream nucleic acid molecule encoding a transgenic protein and directs secretion. The leader sequence may be the native human leader sequence, an artificially-derived leader, or may obtained from the same gene as the promoter used to direct transcription of the transgene coding sequence, or from another protein that is normally secreted from a cell.
Milk-producing cell - A cell (e.g., a mammary epithelial cell) that secretes a protein into milk.
Milk-specific promoter - A promoter that naturally directs expression of a gene in a cell that secretes a protein into milk (e.g., a mammary epithelial cell) and includes, for example, the casein promoters, e.g., α-casein promoter (e.g., alpha S-1 casein promoter and alpha S2-casein promoter), β-casein promoter (e.g., the goat beta casein gene promoter (DiTullio, BIOTECHNOLOGY 10:74-77, 1992), γ-casein promoter, and κ-casein promoter; the whey acidic protein (WAP) promoter (Gorton et al., BIOTECHNOLOGY 5: 1183-1187,1987); the β-lactoglobulin promoter (Clark et al., BIOTECHNOLOGY 7: 487-492, 1989); and the α-lactalbumin promoter (Soulier et al., FEBS LETTS. 297:13, 1992). Also included are promoters that are specifically activated in mammary tissue and are thus useful in accordance with this invention, for example, the long terminal repeat (LTR) promoter of the mouse mammary tumor virus (MMTV).
Nuclear Transfer - This refers to a method of cloning wherein the nucleus from a donor cell is transplanted into an enucleated oocyte.
Operably Linked - A gene and one or more regulatory sequences are connected in such a way as to permit gene expression when the appropriate molecules (e.g., transcriptional activator proteins) are bound to the regulatory sequences.
Ovine - Of or relating to or resembling sheep.
Parthenogenic - The development of an embryo from an oocyte without the penetration of sperm.
Pharmaceutically Pure - This refers to transgenic protein that is suitable for unequivocal biological testing as well as for appropriate administration to effect treatment of a human patient. Substantially pharmaceutically pure means at least about 90% pure.
Porcine - of or resembling pigs or swine.
Promoter - A minimal sequence sufficient to direct transcription. Also included in the invention are those promoter elements which are sufficient to render promoter-dependent gene expression controllable for cell type-specific, tissue-specific, temporal-specific, or inducible by external signals or agents; such elements may be located in the 5' or 3' or intron sequence regions of the native gene.
Recombinant - refers to a nucleic acid sequence which is not naturally occurring, or is made by the artificial combination of two otherwise separated segments of sequence. This artificial combination is often accomplished by either chemical synthesis means, or by the artificial manipulation of isolated segments of nucleic acids, e.g., by genetic engineering techniques. Such is usually done to replace a codon with a redundant codon encoding the same or a conservative amino acid, while typically introducing or removing a sequence recognition site. Alternatively, it is performed to join together nucleic acid segments of desired functional polypeptide sequences to generate a single genetic entity comprising a desired combination of functions not found in the common natural forms. Restriction enzyme recognition sites are often the target of such artificial manipulations, but other site specific targets, e.g., promoters, DNA replication sites, regulation sequences, control sequences, or other useful features may be incorporated by design. A similar concept is intended for a recombinant, e.g., a non-glycosylated or glycan-modified transgenic protein according to the instant invention.
Therapeuticany-effective amount - An amount of a therapeutic molecule or a fragment thereof that, when administered to a patient, inhibits or stimulates a biological activity modulated by that molecule.
Transformed cell or Transfected cell - A cell (or a descendent of a cell) into which a nucleic acid molecule encoding desired protein of the invention related has been introduced by means of recombinant DNA techniques. The nucleic acid molecule may be stably incorporated into the host chromosome, or may be maintained episomally.
Transgene - Any piece of a nucleic acid molecule that is inserted by artifice into a cell, or an ancestor thereof, and becomes part of the genome of the animal which develops from that cell. Such a transgene may include a gene which is partly or entirely exogenous (i.e., foreign) to the transgenic animal, or may represent a gene having identity to an endogenous gene of the animal.
Transgenic -Any cell that includes a nucleic acid molecule that has been inserted by artifice into a cell, or an ancestor thereof, and becomes part of the genome of the animal which develops from that cell.
Transgenic Organism - An organism into which genetic material from another organism has been experimentally transferred, so that the host acquires the genetic information of the transferred genes in its chromosomes in addition to that already in its genetic complement.
Ungulate - of or relating to a hoofed typically herbivorous quadruped mammal, including, without limitation, sheep, swine, goats, cattle and horses.
Vector - As used herein means a plasmid, a phage DNA, or other DNA sequence that (1) is able to replicate in a host cell, (2) is able to transform a host cell, and (3) contains a marker suitable for identifying transformed cells.

According to the present invention, there is provided a method for the production of a transgenic antibody of interest, and variants thereof, the process comprising expressing in the milk of a transgenic non-human placental mammal a transgenic antibody construct that has a modified sugar profile and is amenable to the modification of its glycosylation pattern to improve certain parameters of its performance as a therapeutic agent or as a treatment for a variety of disease conditions. The term "treating", "treat" or "treatment" as used herein includes preventative (e.g., prophylactic) and palliative treatment.

According to the current invention, two general forms of agonistic anti-CD137 antibody are contemplated a form expressed as a aglycosylated form and a second glycosylated form both produced by recombinant caprines or other mammalian "bioreactor." As would be expected, the primary difference between the two forms of the antibody of interest is their glycosylation state, though according to the current invention both are bioactive there are observed differences in their effectiveness profile for specific therapeutic applications. In a preferred embodiment of the current invention the aglycosylated form of the 4-1BB antibody stimulates the EMH through FC cross-linking causing a secondary cytokine cascade causing a prolongation of life of animals carrying life-threatening cancers.

### Working with Murine Antibodies

Therapeutic mouse mAbs that require repeated administration for a full clinical effect are unsuitable for human use because the HAMA response neutralizes the antibody, clears it quickly from the circulation and, in the worst case, induces serious allergic hypersensitivity. Several strategies have been developed to replace most of the murine Ig sequences with human sequences, resulting in fewer side effects while retaining efficacy. The HAMA response may not be a serious problem with anti-CD137 because of the potential inhibitory effects of anti-CD137 on antibody production. Therefore, the most cost effective strategy for developing a human therapeutic mAb is to replace the murine heavy chain (H) and light chain (L) constant regions (C_{H} and C_{L}, respectively) with human regions so that the resulting chimeric antibody is comprised mostly of human IgG protein sequence except for the antigen-binding domains. This is the strategy used for Rituxan® (Rituximab anti-human CD20, Genentech), the first monoclonal antibody approved in the U.S. to treat non-flodgkin lymphoma. By some estimates, providing therapeutic mAbs with human C_{H} and C_{L} sequences should eliminate approximately 90% of the immunogenicity of murine antibody proteins.

An alternative strategy for developing a clinical mAb product is to produce antibody in transgenic mice in which the entire native Ig repertoire has been replaced with human Ig genes. Such mice produce fully human antibody proteins. In this way a chimeric, humanized or fully human antibody is produced as one of several preferred embodiments of the current invention. However, both this antibody and a chimeric one would retain their effector function and would be useful in the treatment of cancer and cancerous lesions. The proposed chimeric antibody embodiment of the current invention retains the original murine variable (antigen-binding) sequences and hence should retain its binding and functional properties.

### Comparison of Aglycosylated Forms of Recombinant IgG to Glycosylated Forms

Glycosylation is a post-translational modification that can produce a variety of final protein forms in the natural state. IgG molecules are glycosylated at the ASN₂₉₇ residue of the CH2 domain, within the Fc region. One important aspect of purifying recombinant proteins from any expression system is demonstrating that the final product has a glycosylation pattern that is comparable to the native protein, but this is difficult given the natural micro-heterogeneity in carbohydrate structures. Failure to achieve comparable glycosylation during protein expression could lead to the addition of specific carbohydrate processing steps during purification, which would add complexity and cost. Having an aglycosylated IgG product that lacked carbohydrates would simplify purification and allow us to develop a more efficient and consistent high-yield process to produce clinical-grade preparations. Other studies with genetically engineered mAbs have shown that glycosylated and aglycosylated IgG's have comparable binding to Fc receptors and Protein A *in vitro* and comparable circulating half-lives *in vivo.* However, according the current invention we have also noted a variance in the function of glycosylated and aglycosylated antibodies. That is, it appears that the aglycosylated form is more successful in protecting against physiological conditions such as leukemia and lymphoma as opposed to a glycosylated embodiment of the current invention that demonstrates effectiveness against solid tumors, cancerous BMT conditions and cancerous lesions.

### Development of an Agonistic Anti-Human CD137 mAb for Use as a Immunotherapeutic Treatment for Cancer

An agonistic anti-human CD137 mAb has been developed for testing as a potential immunotherapeutic treatment for cancer. Antibodies against murine CD137 were raised in rats that were immunized with a fusion protein consisting of the extracellular domain of murine CD137 and human Ig constant (C) region. The leading candidate reagent, clone 2A, is an IgG₂ₐ protein that has been well characterized *in vitro* and *in vivo,* as described in the background section above. The antibody so produced was a murine anti-human CD137 mAb that specifically recognizes human CD137 and does not cross-react with murine CD137. The leading candidate reagent, designated Clone GW, binds specifically to transfected Chinese hamster ovary (CHO) cells expressing human antibody.

### Functional Antibody Molecules

Antibodies are covalent heterotetramers comprised of two identical Ig H chains and two identical L chains that are encoded by different genes. Formation of a mature functional antibody molecule requires that the two proteins must be expressed in the same cell at the same time in stoichiometric quantities and must self-assemble with the proper configuration. According to the current invention the mice and goats expressing mature functional antibodies by co-transfecting separate constructs containing the H and L chains. It is important that both transgenes integrate into the same chromosomal site so that the genes are transmitted together to progeny and protein expression is jointly regulated in individual mammary duct epithelial cells that produce milk proteins. In practice, these requirements have been met in transgenic mice and goats.

### DESIGN & METHODS

### Transgenic Production Methods:

Transgenic animals, capable of recombinant antibody expression, are made by co-transfecting separate constructs containing the heavy and light chains. Glycosylated and aglycosylated versions were made by site-directed mutagenesis. According to the current invention two versions of each construct have been prepared.

According to a preferred embodiment of the current invention, the anti-human CD137 antibodies of the invention were developed and tested to determine their anti-tumor activity. Two xenograft human tumor models were used: ovarian carcinoma in NOD-SCID mice and EBV-induced B lymphoma in SCID mice.

To produce primary cell lines containing the chimeric anti-human CD137 construct for use in producing transgenic goats by nuclear transfer. The heavy and light chain constructs were transfected into primary goat skin epithelial cells, which were clonally expanded and fully characterized to assess transgene copy number, transgene structural integrity and chromosomal integration site. Several cell lines were chosen for use in generating transgenic goats.

### Cloning And Sequencing The Heavy And Light Chain Genes For Anti-Human CD137.

According to a preferred method of the current invention the inventors have constructed a variety of transgene expression vectors containing human constant region sequences for the four major IgG subclasses. These vectors also carry the goat beta-casein promoter and other 5' and 3' regulatory sequences that are used to ensure mammary-specific transgene expression. The chimeric antibody variant of the current invention is constructed by inserting the variable region sequences of the mouse anti-human CD 137 into the constructs developed for the current invention. The first step is to clone and sequence the amino termini of the anti-CD137 H and L chains to identify the murine sequences corresponding to the antibody variable regions.

The inventors have also assembled a collection of oligonucleotides that represent sequences from the 5' coding region of various families of murine immunoglobulins. These sequences were used individually as 5' primers for polymerase chain reaction (PCR) to amplify cDNA prepared from hybridoma RNA, and the resulting PCR products were cloned and sequenced. The 3' PCR primers were prepared from the known sequences of the constant regions. These PCR primers did include appropriate restriction endonuclease sites so that the resulting amplified sequences were inserted into our expression vectors. These sequences were inserted into the constructs to produce genes encoding chimeric proteins. The methods used for the genetic engineering of antibody proteins are known. The methods used to clone and sequencing the anti-CD137 antibody gene variable regions included the following steps:
**1. Make cDNA from hybridoma RNA. RNA were prepared** from the hybridoma by standard methods and cDNA were prepared by reverse transcription with a commercially available kit of reagents (Reverse Transcription System, Promega, Madison, WI).
**2. Amplify cDNA by PCR** with primers based on known sequences from the amino terminus of the V_{H} and V_{L} regions from several well characterized murine monoclonal antibodies.
**3. Amplify the variable region sequences** by inserting the PCR-generated sequences into cloning vectors with the neomycin resistance (neo^{R}) selectable marker and isolating neo^{R} colonies.
**4. Sequence H and L chain cDNA** prepared from approximately 6 colonies to determine the consensus sequence for each variable region. It were important to ensure that no mutations have been introduced into the sequences from PCR artifacts. DNA sequencing were performed on a fee-for-service basis by SequeGen, Co. (Worcester, MA).
**5. Sequence the H and L proteins** isolated from the hybridoma supernatant and compare the actual protein sequences to the deduced protein sequences derived from the gene sequences. This step did confirm that the cloned genes encode functional antibody chains. Protein sequencing were performed on a fee-for-service basis by Cardinal Health (San Diego, CA).

### The Production of Separate chimeric IpG heavy and light chain constructs for chimeric anti-human CD137.

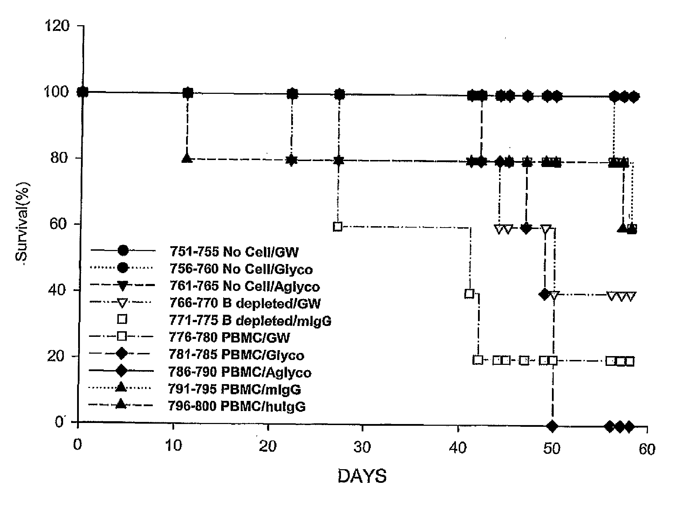

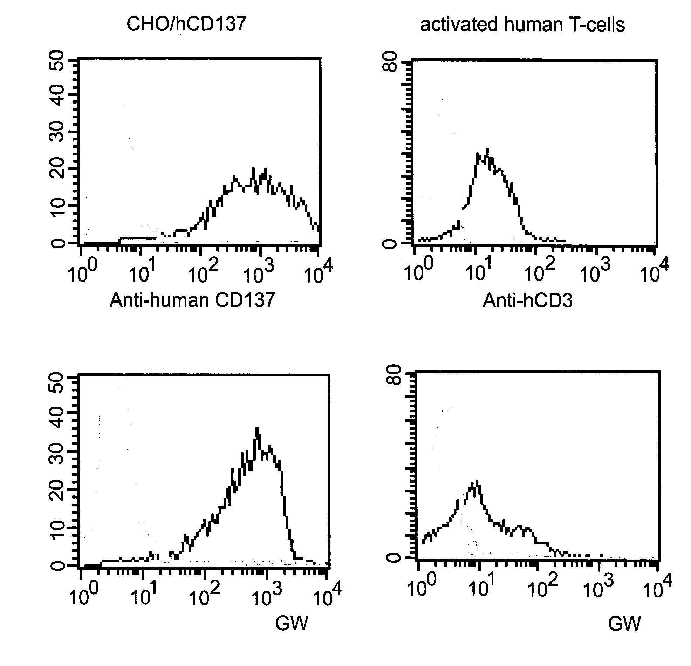

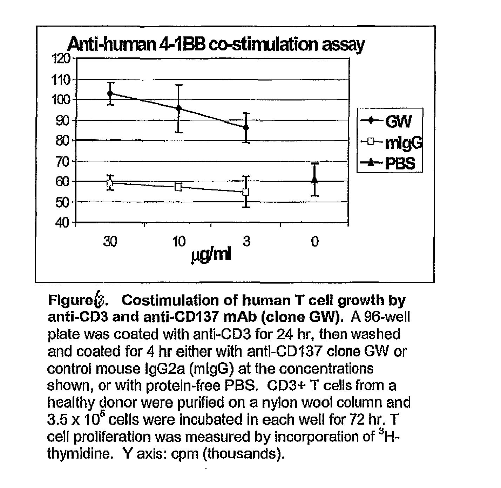

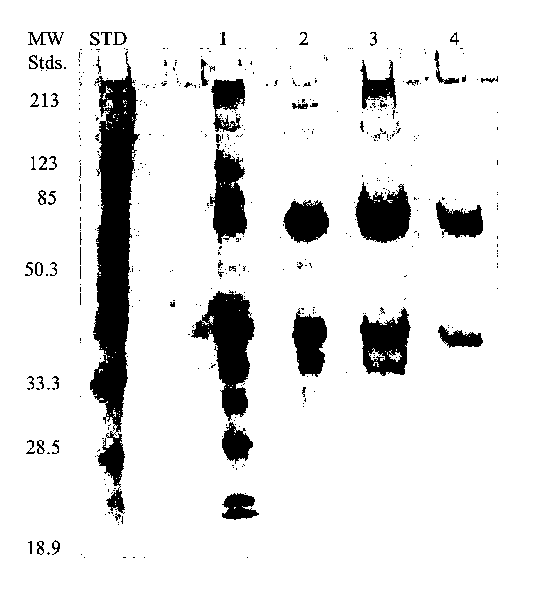

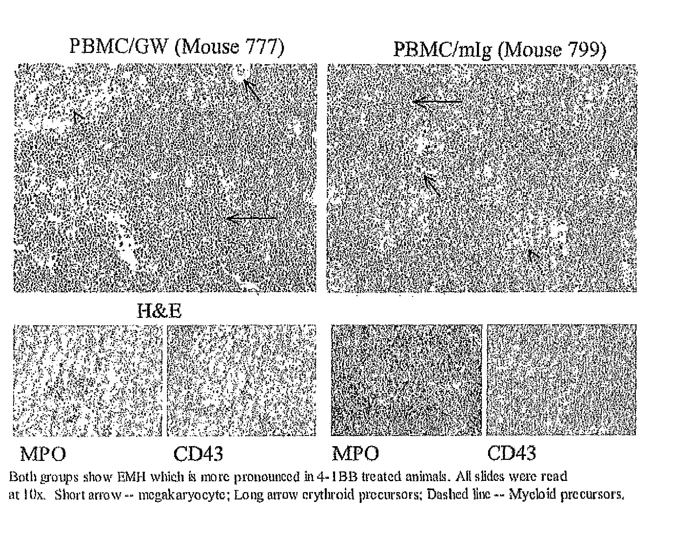

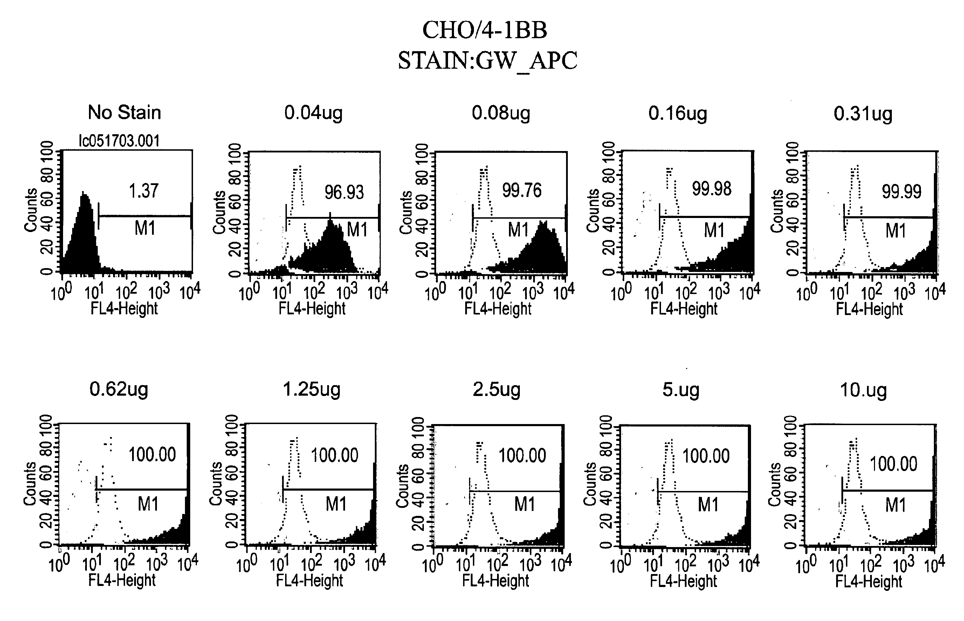

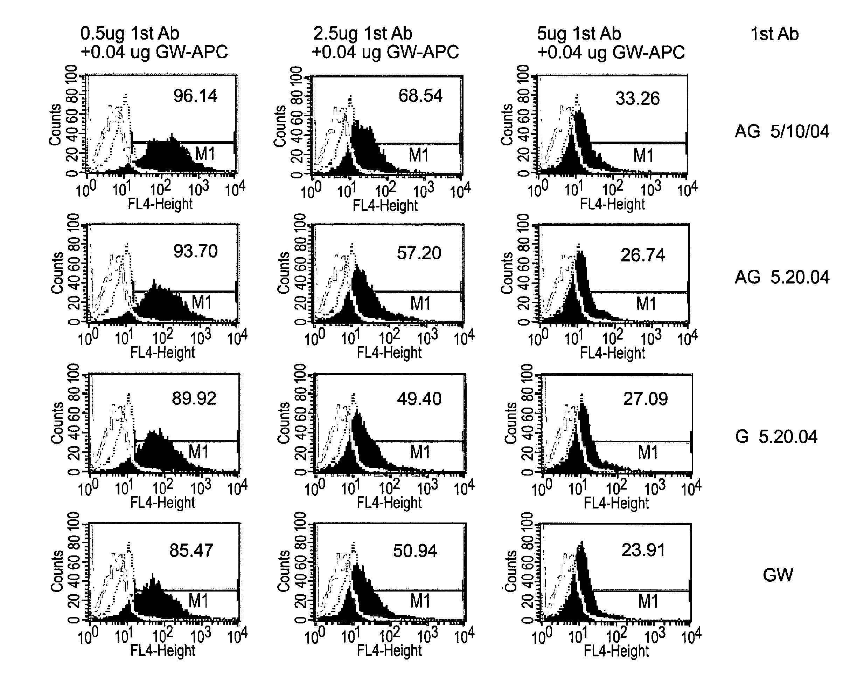

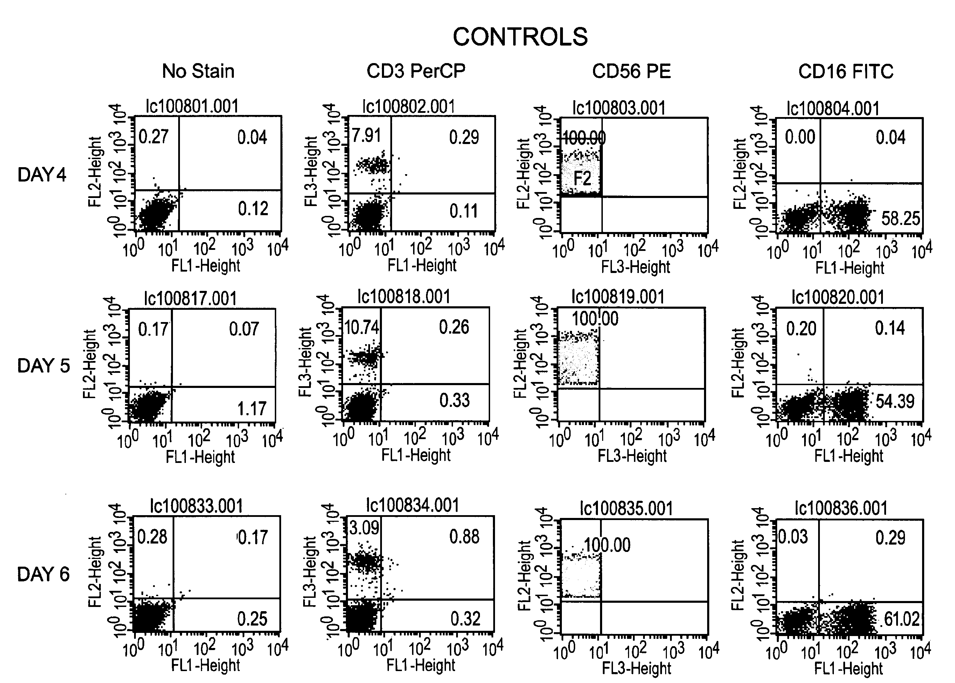

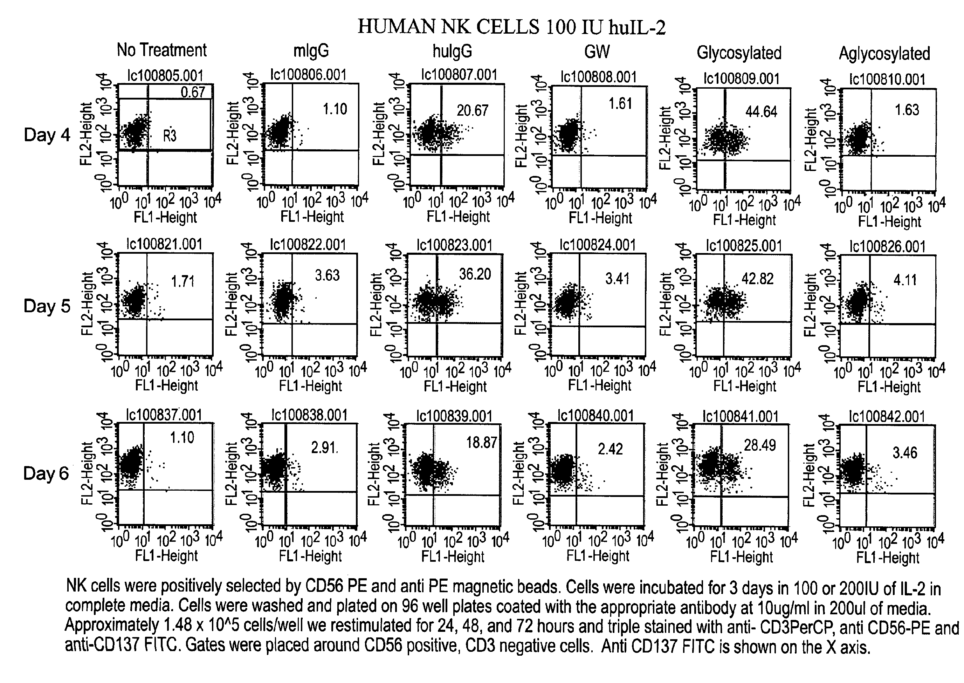

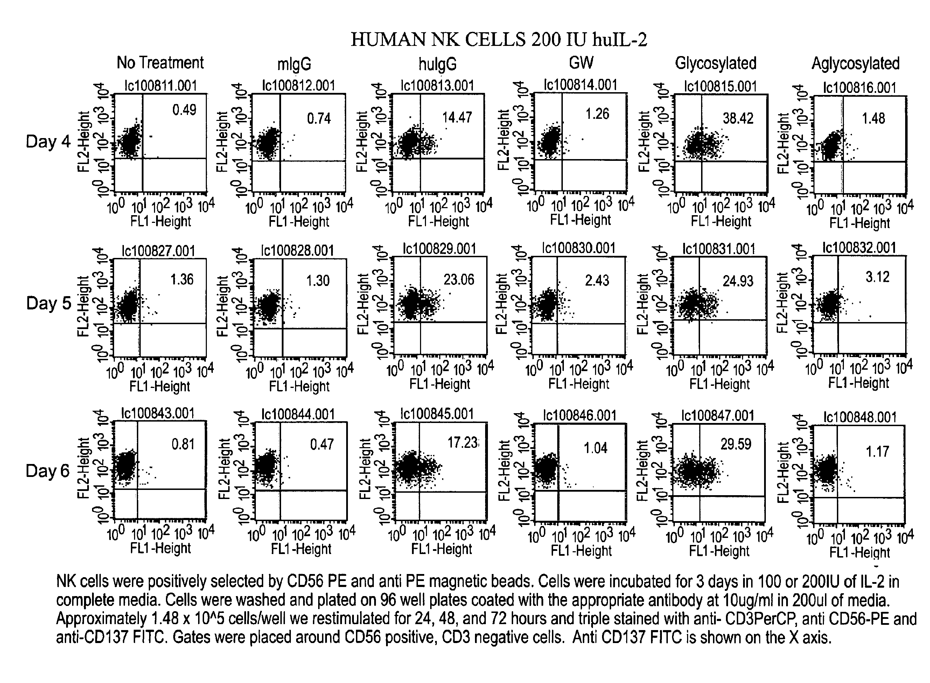

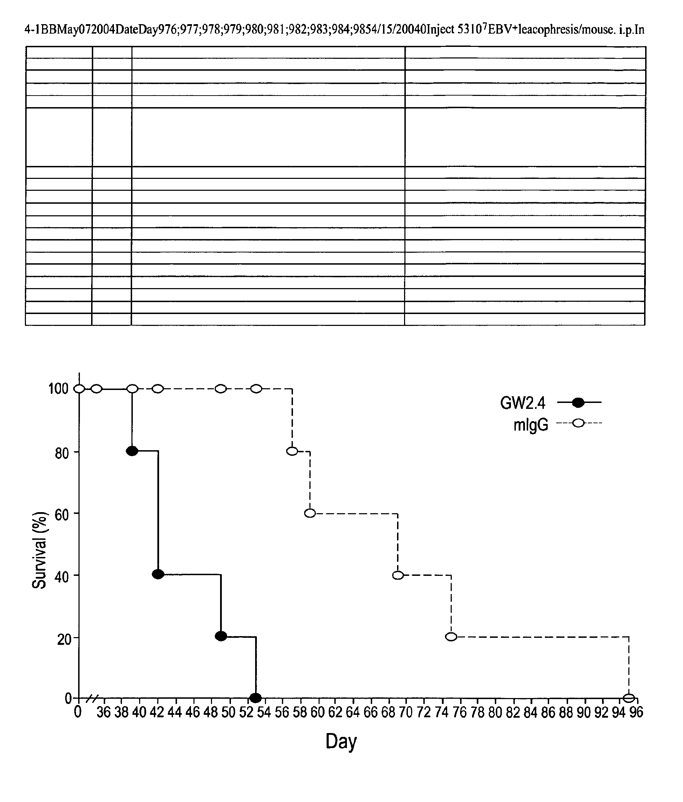

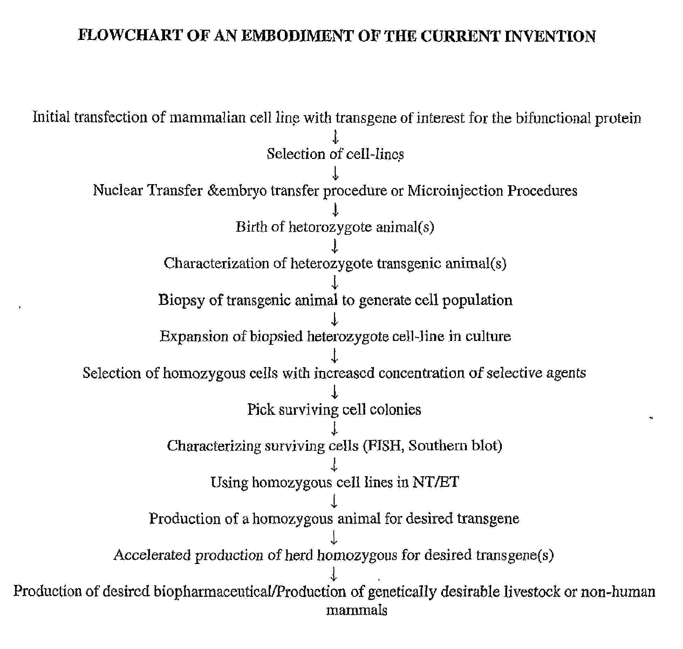

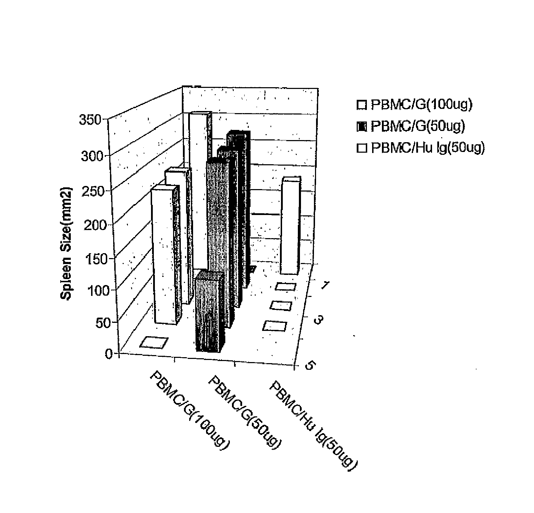

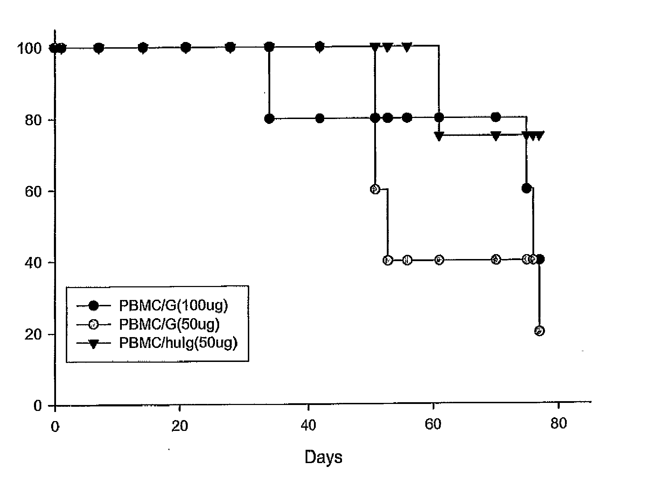

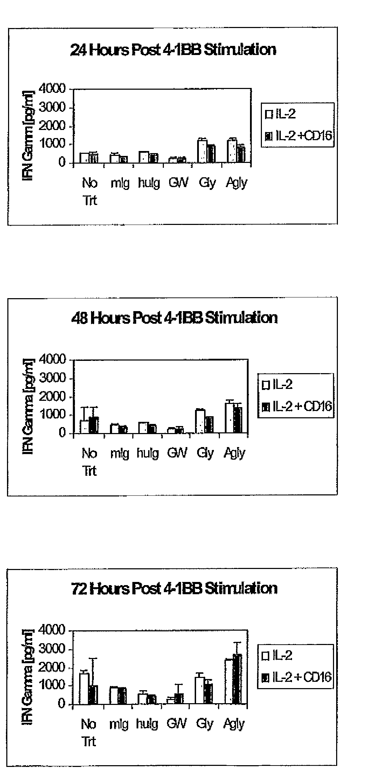

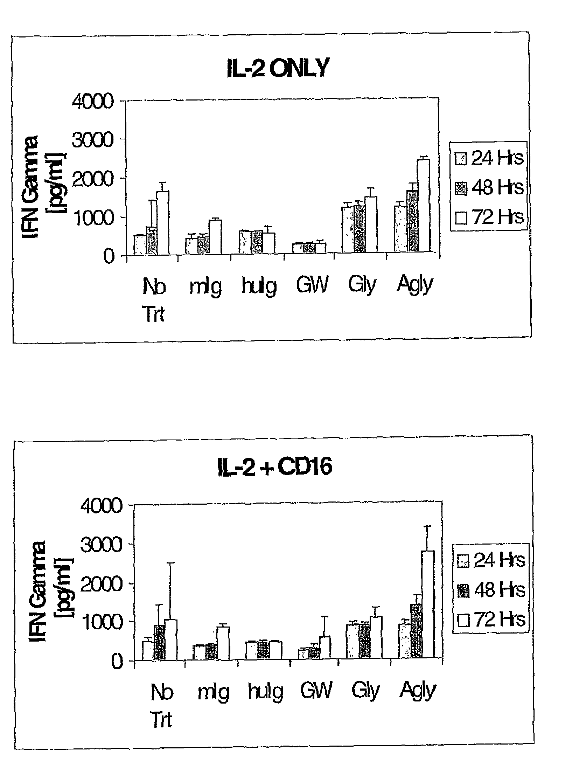

The murine anti-CD137 variable region sequences obtained according to the methods provided above were used to replace human variable region sequences in existing human IgG₁ expression vectors to produce chimeric transgene constructs, as illustrated in Figure 5. The antibody expression vectors utilized contained the necessary IgG₁ H gene in its native glycosylated form. The IgG₁ glycosylation site is an Asn residue at position 297 in the CH2 domain. Also produced was an aglycosylated form of the IgG₁ H chain by altering Asn₂₉₇ to Gln₂₉₇ by site specific mutagenesis. This did give us three constructs: L chain, glycosylated H chain and glycosylated H chain.

Two forms of each construct were prepared for testing and for the generation of transgenic animals. The constructs were used in transient transfection studies to test bioactivity of the genetically engineered chimeric protein.

The constructs used for transgenic animal development contained the goat β-casein promoter and other 5' and 3' regulatory sequences that are used to ensure high level mammary-specific transgene expression. Because of the cross-species recognition of the promoter and other regulatory elements, the same construct was used to generate transgenic mice and goats.

IgG₁ H and L chain expression vectors/gene constructs containing these two sets of regulatory elements already exist. According to the current invention the structural integrity of the constructs by restriction mapping was confirmed.

### Production of Aglycosylated H Chain.

In a preferred embodiment the CH2 domain of the IgG₁ H chain gene was altered Asn₂₉₇ ⇒Gln₂₉₇ in the CH2 domain by site specific mutagenesis with the QuikChange^{®} II XL Site-Directed Mutagenesis Kit (Stratagene, La Jolla, CA) using appropriate oligonucleotides.

### Production of chimeric V_{H} and V_{L} constructs.

For purposes of the current invention human variable region sequences in existing IgG₁ expression vectors were used along with the murine variable region sequences to produce a chimeric humanized antibody.

### Confirmation of structural integrity of the constructs.

Each construct were evaluated by restriction mapping via Southern blot analysis after cleavage with specific restriction endonucleases to confirm that the transgenes are regulatory elements remain structurally intact. The constructs completed were used to make transiently transfected cells and transgenic animals according to the current invention.

### Expressing Chimeric Anti-Human CD137 In Transiently Transfected 293T Cells With Normal Binding Affinity And Specificity.

The chimeric anti-human CD137 antibody were expressed in a transient transfection system so that it could be confirmed that its binding affinity and specificity are comparable to the original murine monoclonal antibody. It was important to test the chimeric mAb to confirm that it retains the binding and functional properties of the original mAb. Myeloma cells can express "irrelevant" Ig proteins that are unrelated to the designated mAb, and mutations were introduced by the PCR amplification step. As a result, the cloning process can produce sequences for antibodies that lack the desired binding and functional characteristics. Hence, supernatants from transfected cells expressing both the glycosylated and aglycosylated chimeric antibody preparations were compared with the chimeric and original murine antibody preparations in the same *in vitro* assays had been used to characterize the anti-CD137 antibody originally:
● Binding specificity assessed by flow cytometric analysis of anti-CD137 binding to activated human T-cells and to two lines of transfected cells expressing CD137 on their surface: CHO/CD137 and P815/hCD137.
● Binding affinity were evaluated by measuring the ability of chimeric CD 137 to inhibit binding of the original monoclonal antibody in a semi-quantitative competitive binding assay.
● Dose-dependent enhancement of human T-cell proliferation and cytokine production by immobilized anti-CD137.

### Transient Transfection.

Chimeric L chain constructs were co-transfected with either glycosylated or aglycosylated H chain constructs into 293T cells, a human renal epithelial cell line that has been transformed by the adenovirus E1.A gene product. The 293T subline also express SV40 large T antigen, which allows episomal replication of plasmids containing the SV40 origin and early promoter region. Transfections were carried out by the standard calcium phosphate precipitation method. After transfection, cells were washed free of calcium phosphate and cultured for 4 days. Supernatant did collected and either tested directly or separated over a Protein A column to isolate IgG.

### Binding Specificity And Affinity.

Anti-CD137 binding specificity and affinity were tested against CHO/CD137 and activated human T-cells. Freshly isolated human peripheral blood T-cells were activated for 24 hr in the plates coated with anti-CD3 and anti-CD28 monoclonal antibodies (PharMingen, San Diego, CA). Cells were harvested and stained with anti-CD137 or an isotype-matched control mAb, in the presence or absence of purified human CD137Ig fusion protein, and then with FITC-conjugated goat anti-human IgG1 antibody. Stained cells were fixed in 1% paraformaldehyde and analyzed by flow cytometry. Binding affinity were measured semi-quantitatively by the dose range over which chimeric anti-CD137 inhibits binding by the original GW mAb, compared to control IgG. The glycosylated and aglycosylated chimeric preparations were compared to the original GW mAb.

### Dose-dependent Co-stimulation of T-cell growth and cytokine production by immobilized anti-CD137.

A co-stimulation assay for anti-CD137 were performed. Briefly, fresh human T-cells that have been purified on a nylon-wool column were stimulated with plate-bound anti-CD3 and various concentrations of chimeric anti-CD 137. Typical concentrations used to test the original GW mAb ranged from about 1 to 25 µg/ml. ³H-thymidine was added during the last 15 hr of the 3-day culture. Radioactivity in harvested cells were measured with a MicroBeta TriLux liquid scintillation counter (Wallac). The glycosylated and aglycosylated chimeric preparations were compared to the original GW mAb with plate-bound isotype-matched IgG as a control. In addition, the supernatants from these cultures with ELISA were assayed to measure supernatant gamma-interferon, which is selectively stimulated by anti-CD137.

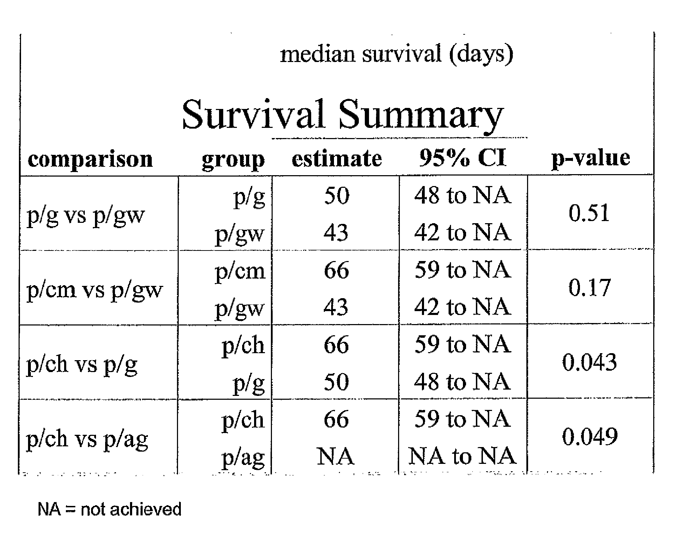

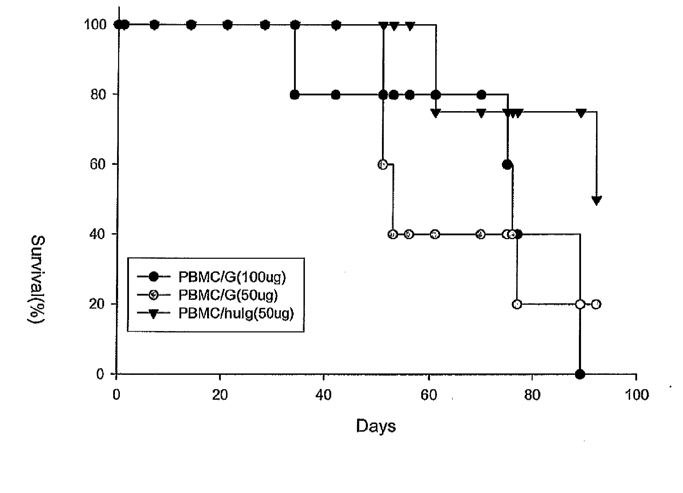

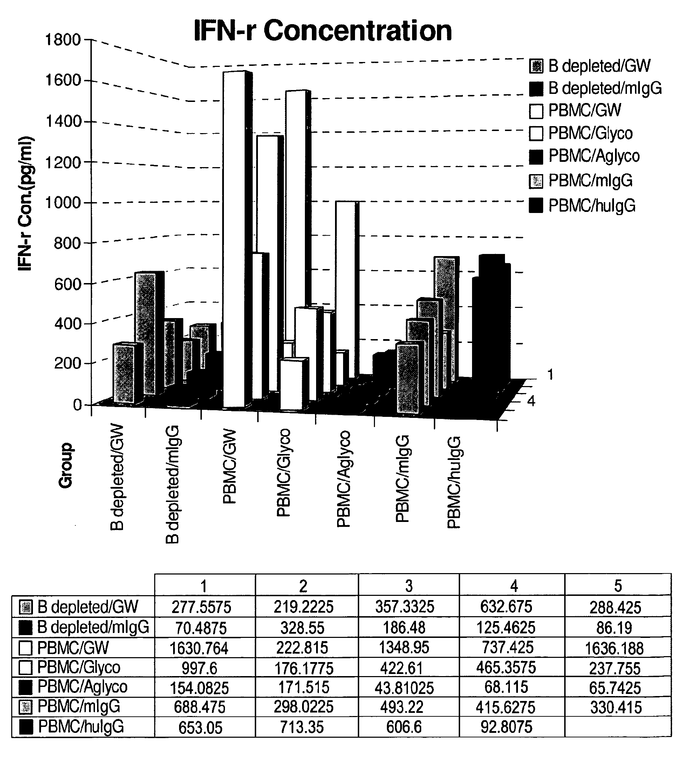

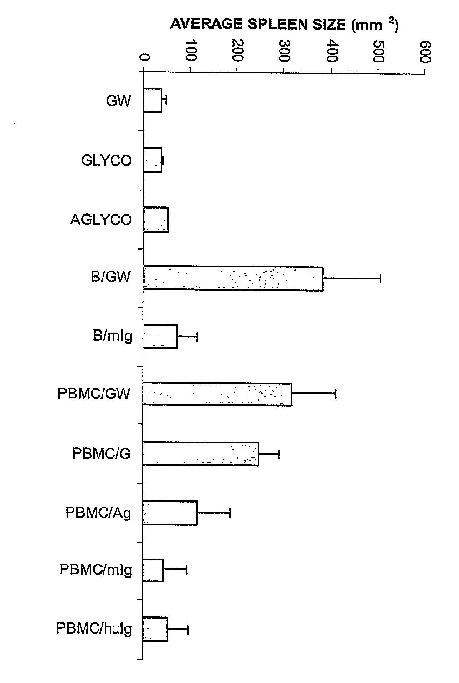

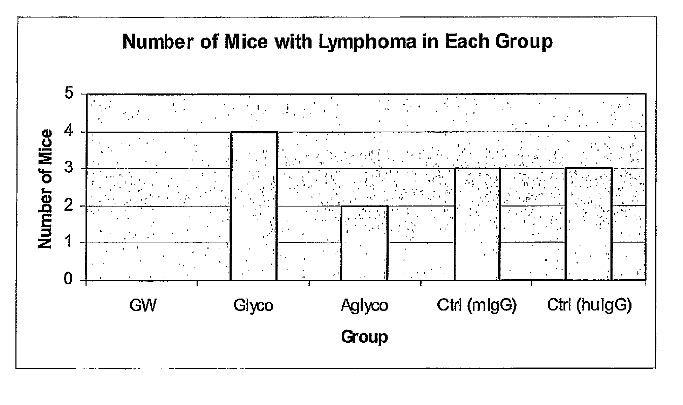

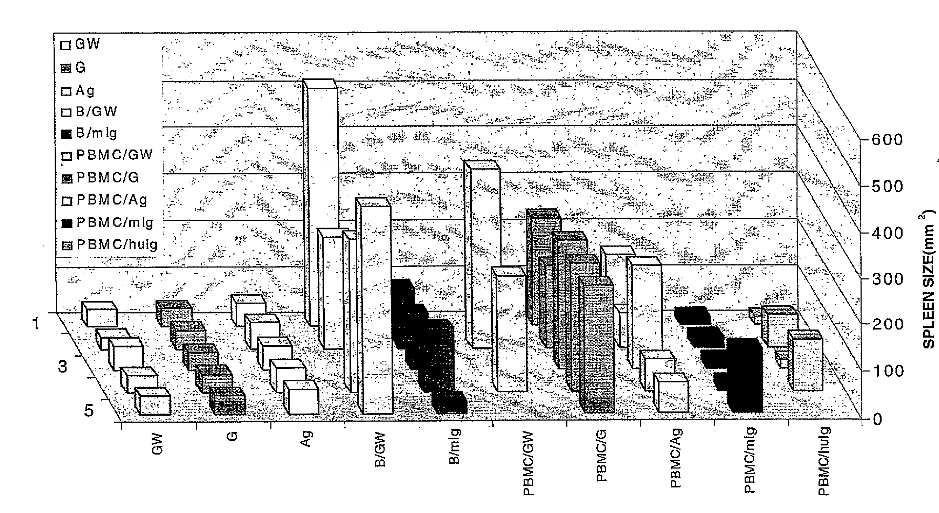

Referring to Figure 34, the method of measuring γ interferon activity was as follows:
Day0 inject cells
Day1: inject Ig(200µg/ml), keep injection weekly.
Day18:Drew blood from tail vein, collect serum. Serum were kept in - 20°C.

### ELISA

The ELISA was performed with Human IFN-r ELISA kit (eBioscience) following the instruction. Capture antibodies were coated to the plate with incubation under 37°C, 4hrs. After wash with TPBSx4, blocking solution was applied and incubated 30min under RT. After wash with TPBSx4, standard were add to the plate with the starting concentration of 500pg/ml. Serum were diluted 1/5 with blocking solution and add to the plate, then stayed 4°C overnight Detect antibodies were add after plate wash and incubated 1 hour under RT. Then developed with TMB and stopped by ²N H₂SO₄. The plate was read by MRX revelation plate reader.

Successful demonstration that the chimeric anti-CD137 antibody is comparable to the original GW mAb in antibody binding specificity and affinity and dose-dependent T-cell stimulatory properties confirmed the utility of the current invention and justified the production of transgenic animals for larger scale antibody production. If both the glycosylated and aglycosylated chimeric antibody preparations show appropriate binding specificity and affinity, then both were used to generate transgenic mice.

### Generation Of Transgenic Animals Expressing Both Glycosylated And Aglycosylated Chimeric Anti-Human CD137 In Their Milk.

According to the current invention both glycosylated and aglycosylated chimeric and humanized antibodies to the anti-human CD 137 antibodies have been produced. After the purification of sufficient quantities of antibody from milk to test the bioactivity it was found that though they were essentially produced at identical levels, the activity profiles of the two forms differed. More importantly their activity against given specific types of cancers varied with each version offering a variable level of activity vis-à-vis the other form.

Transgene constructs for the chimeric antibodies were used to generate transgenic mice and gosts to test secretion and bioactivity of the chimeric anti-CD137 preparations. Transgenic animals produce mature antibodies by introducing a 1:1 mixture of H chain and separately L chain constructs. The L chain construct were combined with either the glycosylated or aglycosylated H chain construct. Specifically, the relative and absolute levels of bioactive product in milk was measured by Western blot analysis and measure antibody binding *in vitro.*

The most practical strategy for testing the feasibility of the inducible systems in transgenic mice was to evaluate transgenic protein expression in the milk of first-generation (F₁ mice. It has been determined that in some transgenic animals, the original transgene constructs integrate into several chromosomes after microinjection, and these chromosomal integration sites segregate into the genome in the following 1 or 2 generations to form stable, homogeneous transgenic animal lines. Therefore, F₁ mice are reasonable models for determining the stability of transgene expression. Moreover, in order for mice to lactate, they must mature (which takes about 2 months), mate and produce offspring. After analysis it was determined that the secretion levels were stable and the construct used was effective.

### Transgenic Mice.

Linear DNA from each construct prepared is purified by CsCl gradient followed by electroelution, and transgenic mice were generated by pronuclear microinjection. Transgenic founder animals are identified by PCR analysis of tail tissue DNA and relative copy number were determined using Southern blot analysis. The goal was to produce 10 transgenic first-generation transgene-bearing "founder" (F₀) females from each construct (glycosylated and aglycosylated). This allowed for variations in expression due to possible chromosomal rearrangements and position-dependent variegation that were generated by transgene integration. These F₀ mice were mated at maturity to initiate lactation. Their milk were analyzed on Western blots developed with goat anti-human-Fc antibody to identify mice that secrete structurally intact Chimeric antibodies bearing the human C_{H} region.

The best founders, defined as healthy animals with the maximum reasonable expression of antibody in their milk, were then bred to the second (F₂) generation so that enough milk from the F₁ females is collected for antibody testing *in vitro* and *in vivo.*

### Characterization of milk-derived chimeric anti-CD137.

Protein A-purified IgG fractions isolated from pooled milk samples from each line were analyzed *in vitro* to characterize antibody binding specificity and affinity and dose-dependent enhancement of T-cell proliferation. In a preferred embodiment, we compared milk-derived glycosylated and aglycosylated chimeric preparations to the original monoclonal antibody and to the original GW mAb.

Production of healthy transgenic mice with normal growth and reproductive characteristics and reasonable levels (> 1 mg/ml) of bioactive anti-CD137 were the next step in establishing the feasibility of this approach to producing an immunomodulator to treat solid tumors. Production in mice with a given construct has been a precursor to work in large scale production species such as caprines or bovines. That is, according to the current invention, success with the production of transgenic mice indicates production success on a larger scale for the production of anti-human CD137. In the instant case, production and characterization of chimeric anti-CD137 led to testing of one or more of these preparations in a mouse model to demonstrate anti-tumor activity *in vivo.* Both the glycosylated and aglycosylated chimeric antibody constructs thereafter resulted in the production of transgenic goats expressing anti-CD137 in their milk.

### Chimeric Anti-Human CD137 as a Preclinical Model of Anti-Tumor Activity.

A second major hurdle in the clinical transition of co-stimulatory approaches to cancer immunotherapy is the demonstration of effectiveness of the antibodies in an appropriate model system *in vivo*. According to the current invention two xenograft mouse models for testing the effects of ability of immune modulators to amplify T-cell-mediated immune responses were used:
**1. Human ovarian carcinoma in the NOD-SCID (non-obese diabetic / severe combined immune deficiency) mouse (the "NOD-SCID ovarian carcinoma" model).** Ascites samples from patients with ovarian cancer are fractionated to recover tumor cells, which are injected into the NOD-SCID mice either subcutaneously or intraperitoneally to induce tumors. A lymphocyte-enriched cell fraction from the same patients is injected into the mice after the tumors have become established. These lymphocytes alone are not sufficient to cause significant tumor regression, but treatment with immune modulators can augment the immunological response to tumor. NOD-SCID mice have multiple immune defects, which allow reconstitution with human cancer cells and hematopoietic cells
**2. Spontaneous human Epstein-Barr virus-induced lymphoma in the SCID mouse (the "EBV-LPD" model).** SCID mice are reconstituted with peripheral blood mononuclear cells (PBMC) derived from normal healthy donors that are seropositive for EBV. After injection, the majority of mice (up to 85%) develop a fatal lymphoproliferative disease (EBV-LPD) due to transformation of B cells by EBV virus. After engraftment, human NK cells and T-cells survive for a long period of time and were activated by IL-2 and GM-CSF to prevent the development of EBV-LPD.

Both of these systems provide clinically relevant models for evaluating the bioactivity of chimeric anti-human CD137 antibody in activating T-cells and antitumor immunity. With both models, treatment success were evaluated by the increase in survival and (in one variant of NOD-SCID model) a decrease in solid tumor volume. At this time, the NOD-SCID ovarian carcinoma model has been used to evaluate antitumor effects of various co-stimulatory molecules and mAb. According to the current invention, both models can be used in parallel to test the chimeric anti-CD 13 7 preparations.

One objective of this work was to determine whether the aglycosylated form of the chimeric anti-human CD 137 antibody preparation is bioactive *in vivo.* It was determined that it is.

### Evaluation of anti-CD137 in the NOD-SCID ovarian carcinoma model.

Female NOD-SCID mice (Strain NOD.CB17-SCID, Jackson Laboratory, Bar Harbor, ME) were sublethally irradiated to kill residual non-thymic-derived NK cells and used as described by Dr. Chen. [9] with small modifications. Briefly, ascites fluid from patients with primary ovarian cancer were collected and centrifuged over Ficoll/Hypaque to separate two fractions: tumor cells and a lymphocyte-enriched fraction. A portion of the tumor cells and all of the lymphocytes were cryopreserved. Washed suspensions of tumor cells were injected at doses of 2 x 10⁷ cells in 200 microliters buffered saline into one of two sites on different mice: dorsal subcutaneous tissue, to establish solid tumors, or intraperitoneally (i.p.), to establish an ascites tumor. An ascites aspirate from one patient usually provides enough cells to reconstitute approximately 20 mice. Solid tumor size were measured twice weekly with calipers fitted with a Vernier scale and calculated on the basis of 3 perpendicular measurements.

After about 1 to 2 weeks, when the tumors become palpable, the lymphocyte fraction were thawed and resuspended with an expected recovery of about 80% viable cells. The cell suspension were injected either intravenously (iv) into mice with solid subcutaneous tumors (2 x 10⁷cells) or i.p (5 x 10⁶ cells) into mice with ascites tumors. Mice received an i.p. injection of 100 µg to 300 µg of chimeric anti-human CD137 and the same treatment did repeat weekly for three more times. Control mice did receive isotype-matched mAb.

Outcomes were measured as survival/time to death for mice with ascites tumors and by reduction in tumor size for mice with solid tumors. Any mice whose tumors reach a mean diameter of 1 cm were sacrificed for humane reasons, in accordance with IACUC guidelines. All mice were sacrificed at the end of the experiment. In addition, some mice with either solid or ascites tumors were assayed to measure the cytolytic activity of their tumor-specific cytotoxic T lymphocytes (CTLs). Briefly, 7 to 10 days after the second antibody treatment, animals were sacrificed and lymphocytes were harvested from tumor-draining lymph nodes. The lymphocytes were restimulated *in vitro* with irradiated carcinoma cells from the original donor. After 4-6 days in culture, the stimulated cells were used as effectors in a standard 4-hour ⁵¹Cr release assay against tumor target cells. T-cells whose responsiveness was augmented *in vivo* by anti-CD137 should kill the target cells more effectively than T-cells treated with isotype-matched control antibody. The survival of the mice were analyzed by the log rank test.

### Evaluation of anti-CD137 in the EBV-LPD model.

This model assay system was used essentially as provided in the prior art. Briefly, normal healthy donors who are EBV seropositive and HIV seronegative, and provide informed consent under a then-current IRB protocol at the Mayo Clinic, did undergo leukophoresis. PBMCs were separated on a Ficoll/Hypaque gradient (Sigma), washed and injected i.p. into SCID mice at a dose of 5 x 10⁷ cells/mouse in 0.5 ml PBS. SCID mice were treated with weekly injections of anti-AsialoGM-1 antiserum to deplete their natural killer (NK) cells and increase the rate of engrafting. Within 6 weeks of PBMC injection, untreated mice usually develop B-cell lymphomas and begin to die. In previous studies, approximately 81% of mice that received human PBMC were successfully engrafted, as established by detection of circulating human Ig by ELISA. Only successfully engrafted mice were used for these studies.

Starting at 4 weeks after PBMC injection, mice received weekly 3 i.p. injections of 100-300 µg of either anti-CD137 test preparation or an isotype-matched negative control (as described above). Mice that receive low-dose GM-CSF plus IL-2 served as positive controls. Outcomes were measured as survival and expansion of human T-cells, detected by flow cytometry analysis using anti-human MHC class I from blood PBMC. The survival of the mice were analyzed by the log rank test.

The chimeric anti-CD137 produced in the milk of transgenic animals was bioactive. Pro-longed survival and increased immune responses in mice after chimeric anti-CD137 treatment also established this recombinant antibody as a potential cancer therapeutic.

The aglycosylated preparation was also found to be bioactive. Previous studies indicated that human NK, T and B cells could survive for at last 2 months after engraftment in NK-cell-depleted SCID mice, and these components probably are sufficient to elicit a successful response to anti-CD137. In the NOD-SCID ovarian carcinoma model, it is sometimes difficult to harvest enough TDLN T-cells to measure their cytolytic activity by 10 days after antibody treatment. If we this is a problem, then we did sacrifice additional mice 14 to 21 days after treatment and recover T-cells from their spleens, which generally provide a higher yield.

Meanwhile, we did prepare for the production of transgenic goats by producing and characterizing cells lines for use as donors in the nuclear transfer procedure. The production of transgenic goats was facilitated by utilizing the same constructs utilized in the development of transgenic mice.

### Production of Transgenic Goats by Nuclear Transfer That Carry the Chimeric Anti-Human CD137 Construct

The inventors used nuclear transfer techniques to generate transgenic goats with pre-defined genetics. The transgene construct was introduced into primary cell lines by a standard transfection method, examples of such techniques include lipofection or electroporation. The recombinant primary cell lines are screened *in vitro* for important characteristics such as transgene copy-number, integrity and integration site before they are used to produce transgenic animals. Nuclear transfer eliminates the problem with transgene mosaicism in the first few generations because all of the animals derived from a transgenic cell line should be fully transgenic. We used female goat skin fibroblasts to make the transfected transgenic cells that served as nuclear donors for nuclear transfer so that all of the resulting offspring were female. This means that milk containing the recombinant protein was obtained directly from F₀ goats. The techniques encompassed by the current invention include: nuclear transfer and pronuclear microinjection. For the current animals and invention nuclear transfer is now the method of choice for most transgenic applications in goats.

### Production Of Skin Fibroblast Lines.

Fibroblasts from fresh goat skin biopsy samples were maintained in primary culture *in vitro.* Briefly, skin samples were minced in Ca⁺⁺-free and Mg⁺⁺- free phosphate buffered saline (PBS), harvested with dilute trypsin in EDTA to recover single cell suspensions and cultured at 37°C. When the cells become confluent they were trypsinized and sub-cultured. Aliquots of cells were cryopreserved in liquid nitrogen.

### Analysis Of Transfected Cell Lines.

Each cell line were characterized by Southern blot analysis with probes specific for the transgene such as beta-casein, chimeric anti-CD137 H and L chain cDNAs to establish the transgene copy number and to look for gross rearrangements. Each cell line also was analyzed by FISH to confirm that there was a single integration site and to determine its chromosomal location, and by cytogenetic analysis to confirm that it has a normal karyotypes. Only primary cultures that are subsequently found to exhibit transgene structural integrity, uniform integration characteristics and normal karyotypes were analyzed further.

### FISH

For Interphase FISH, a few hundred cells from each expanded colony were immobilized on filters and hybridized to amplified transgene-specific digoxigenin-labeled probes. For metaphase FISH, cells were cultured on Lab Tek Chamber slides and pulsed with 5-bromo-2'deoxyuridine (BrdU) to allow for replication banding. Probe binding were detected with FITC-conjugated anti-digoxigenin, and the chromosomes were counterstained with 4',6-Diarnidino-2-phenylindole (DAPI). Images were captured using a Zeiss Axioskop microscope, a Hamamatsu digital camera, and Image Pro-Plus software. Some probes are relatively large and easy to detect by FISH but probes for individual IgG H and L chains, which are encoded by relatively short cDNA sequences, are too small to give good resolution by themselves. These small probes were mixed with sequences from the milk-specific promoter for goat beta-casein. The goat beta casein probe also detects the single copy endogenous goat beta casein gene on chromosome 4, this is a known binding site that does not interfere with interpretation of the results.

### Cytogenetic Analysis.

Cytogenetic analysis of donor transfected fibroblast cell lines was carried out. Transgene probes were labeled with digoxigenin-dUTP by nick translation. Probe binding to the denatured chromosomes were detected either with FITC-conjugated anti-digoxigenin or with horseradish peroxidase-conjugated anti-digoxigenin followed by FITC-conjugated tyramide. Chromosome banding patterns were visualized with DAPI. Goats have 60 chromosomes, all of them acrocentric (having the centromere at one end rather than at or near the middle), which makes them difficult to identify individually. The metaphase spreads for evidence of gross abnormalities such as chromosome loss, duplication or gross rearrangement were inspected.

Cell lines that are used to generate first-generation transgenic goats must be karyo-typically normal and must carry structurally intact chimeric anti-CD137 H and L chain genes along with the beta-casein promoter and other essential regulatory elements.

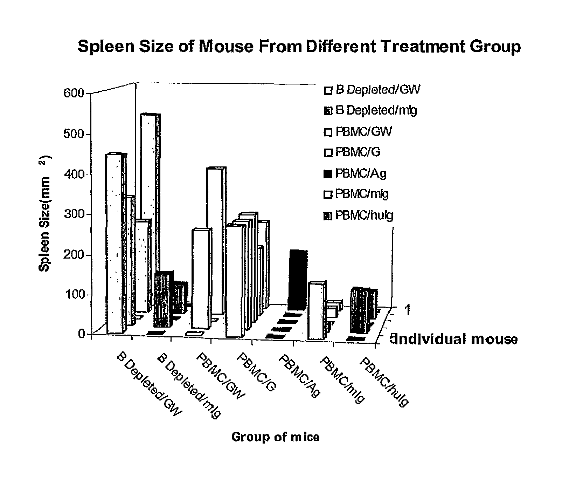

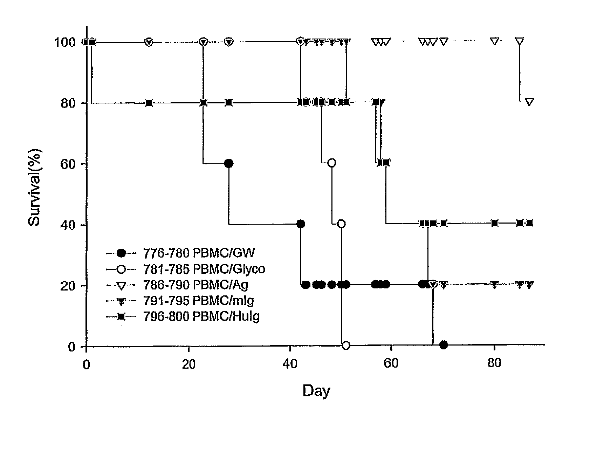

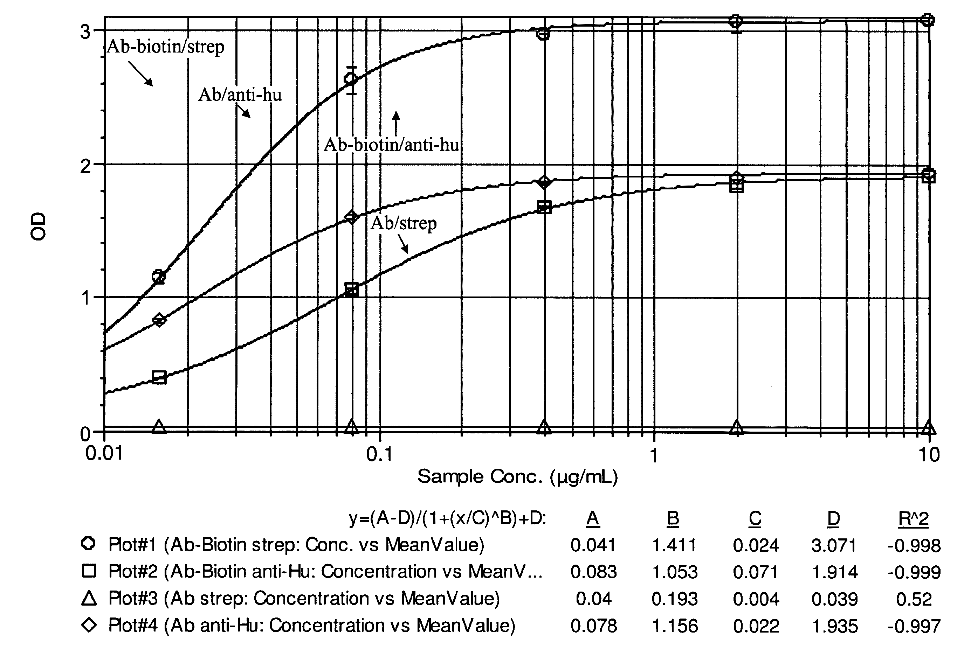

Turning to Figure 3, a biotinylated antibody of interest was tested in an ELISA comprising the following methodology:
Coat: Goat anti-hu IgG (Rockland #609-101-123) diluted 1/1000 in 0.1M NaHCO₃, 100*µ*L/well Plate washed 3 times with plate wash solution
Block: CETS (Casein, EDTA, Tween, PBS), 200*µ*L/well Plate washed 3 times with plate wash solution
Sample: Biotinylated antibody, concentration estimated to be 0.8 mg/mL
   "Non" biotinylated antibody, concentration 1.6 mg/mL Both serially diluted in assay diluent 10 to 0.26*µ*g/mL, 100*µ*g/mL
   (Assay diluent is CETS diluted 1/10 with plate wash solution)
Plate washed 3 times with plate wash solution
Detection Protocol: Goat anti-human H&L-AP (Southern Biotechnology #2060-04 and Roche #605415 pooled) Strep-AP (Southern Biotechnology #7100-04) Each diluted 1/1000 with assay diluent, 100*µ*g/well, Both detects applied to both samples
Plate washed 3 times with plate wash solution Substrate: Liquid PNPP (Cygnus # F008), 100*µ*L/well Stop: 0.1M EDTA (VWR #VW3314-1), 100*µ*L/well
Plate read at 405nm with 490nm background correction

As indicated by Figure 3, the antibody was biotinylated. The Goat anti-human H&L-AP detect bound to both biotinylated and non-biotizxylated antibody. Strep-AP only bound to the biotinylated antibody.

The 4-1BB antibody CD137 produced according to the current invention was cloned and expressed in the milk of several lines of transgenic mice and goats as a genomic "mini-gene." The expression of this gene is under the control of the goat β-casein regulatory elements. Substantial expression of the antibody variants according to the current invention in both mice and goats has been established.

One of the initial targets for imnunotherapeutic use of the current agonistic anti-CD 137 antibody is for use with patients suffering from squamous cell carcinoma of the head and neck.

One of the objectives of the current invention is to establish the production of bioactive anti-human CD137 antibody, an immune modulator that may be effective against solid tumors, in the milk of transgenic animals. CD137 (also called 4-1BB) is a membrane glycoprotein that were induced in several types of lymphoid cells. An agonistic monoclonal antibody (mAb) against murine CD137 shrank mouse tumors in vivo and prevented their recurrence, suggesting that anti-CD137 may be effective against human tumors. The next technical hurdle to clinical translation is to develop a genetically engineered form of the anti-human CD137 that is suitable for clinical use, and to demonstrate that it is effective against human tumors in an appropriate mouse model.

One of the tools used to predict the quantity and quality of the recombinant protein expressed in the mammary gland is through the induction of lactation (Ebert KM 1994; Sato T 1997; Cameos C 2000). The procedure makes it possible to analyze the protein from the early stage of transgenic production rather than that from first natural lactation resulting from pregnancy a year later. Induction of lactation was performed either hormonally or manually. It is unknown whether there are any effects of inducing lactation on the glycosylation of transgenic proteins. It is possible that various lactation procedures, especially hormonally-induced lactation, might affect the transcriptional regulation of glycosyltransferases in mammary gland. Data generated according to the current invention shows that the N-linked oligosaccharides from various lactation samples of cloned animals were similar except for the content ofNeuGc. Carbohydrates in transgenic antibody production from natural lactation contained higher amount of NeuGc than that from other lactation procedures, even though the overall sialic acid content in samples from different lactation was comparable. Likewise, it appears that transgenic proteins produced in the milk of goats are also comprised of a complex mixture of individual protein species. Taken together, these data provide evidence for the feasibility of large scale production of complex glycoproteins from the pooled milk of a herd of transgenic goats derived from a common founder. Obviously, product release specifications would have to be established to ensure that the glycosylation heterogeneity is reproducible, just as is required for CHO cell expression of therapeutic glycoproteins.

### Cloned Animals.

The present invention also includes a method of cloning a genetically engineered or transgenic mammal, by which a desired gene is inserted, removed or modified in the differentiated mammalian cell or cell nucleus prior to insertion of the differentiated mammalian cell or cell nucleus into the enucleated oocyte.

Also provided by the present invention are mammals obtained according to the above method, and the offspring of those mammals. The present invention is preferably used for cloning caprines or bovines but could be used with any mammalian species. The present invention further provides for the use of nuclear transfer fetuses and nuclear transfer and chimeric offspring in the area of cell, tissue and organ transplantation.

Suitable mammalian sources for oocytes include goats, sheep, cows, pigs, rabbits, guinea pigs, mice, hamsters, rats, primates, etc. Preferably, the oocytes were obtained from ungulates, and most preferably goats or cattle. Methods for isolation of oocytes are well known in the art. Essentially, this did comprise isolating oocytes from the ovaries or reproductive tract of a mammal, e.g., a goat. A readily available source of ungulate oocytes is from hormonally induced female animals.

For the successful use of techniques such as genetic engineering, nuclear transfer and cloning, oocytes may preferably be matured *in vivo* before these cells may be used as recipient cells for nuclear transfer, and before they were fertilized by the sperm cell to develop into an embryo. Metaphase II stage oocytes, which have been matured *in vivo,* have been successfully used in nuclear transfer techniques. Essentially, mature metaphase If oocytes are collected surgically from either non-super ovulated or super ovulated animals several hours past the onset of estrus or past the injection of human chorionic gonadotropin (hCG) or similar hormone.

Moreover, it should be noted that the ability to modify animal genomes through transgenic technology offers new alternatives for the manufacture of recombinant proteins optimized for use a therapeutic in humans in terms of their glycan profile. The production of human recombinant pharmaceuticals in the milk of transgenic farm animals solves many of the problems associated with microbial bioreactors (e.g., lack of post-translational modifications, improper protein folding, high purification costs) or animal cell bioreactors (e.g., high capital costs, expensive culture media, low yields). The current invention enables the use of transgenic production of biopharmaceuticals, transgenic proteins, plasma proteins, and other molecules of interest in the milk or other bodily fluid (i.e., urine or blood) of transgenic animals homozygous for a desired gene that then optimizes the glycosylation profile of those molecules.

According to an embodiment of the current invention when multiple or successive rounds of transgenic selection are utilized to generate a cell or cell line homozygous for more than one trait such a cell or cell line were treated with compositions to lengthen the number of passes a given cell line can withstand in *in vitro* culture. Telomerase would be among such compounds that could be so utilized.

The use of living organisms as the production process means that all of the material produced were chemically identical to the natural product. In terms of basic amino acid structures this means that only L-optical isomers, having the natural configuration, were present in the product. Also the number of wrong sequences were negligible because of the high fidelity of biological synthesis compared to chemical routes, in which the relative inefficiency of coupling reactions did always produce failed sequences. The absence of side reactions is also an important consideration with further modification reactions such as carboxy-terminal amidation. Again, the enzymes operating *in vivo* give a high degree of fidelity and stereospecificity which cannot be matched by chemical methods. Finally the production of a transgenic protein of interest in a biological fluid means that low-level contaminants remaining in the final product are likely to be far less toxic than those originating frozen a chemical reactor.

As previously mentioned, expression levels of three grams per liter of ovine milk are well within the reach of existing transgenic animal technology. Such levels should also be achievable for the recombinant proteins contemplated by the current invention.

In the practice of the present invention, non-glycosylated related transgenic proteins are produced in the milk of transgenic animals. The human recombinant protein of interest coding sequences were obtained by screening libraries of genomic material or reverse-translated messenger RNA derived from the animal of choice (such as cattle or mice), or through appropriate sequence databases such as NCBI, genbank, etc. These sequences along with the desired polypeptide sequence of the transgenic partner protein are then cloned into an appropriate plasmid vector and amplified in a suitable host organism, usually *E. coli.* The DNA sequence encoding the peptide of choice can then be constructed, for example, by polymerase chain reaction amplification of a mixture of overlapping annealed oligonucleotides.

After amplification of the vector, the DNA construct would be excised with the appropriate 5' and 3' control sequences, purified away from the remains of the vector and used to produce transgenic animals that have integrated into their genome the desired non-glycosylated related transgenic protein. Conversely, with some vectors, such as yeast artificial chromosomes (YACs), it is not necessary to remove the assembled construct from the vector; in such cases the amplified vector may be used directly to make transgenic animals. In this case non-glycosylated related refers to the presence of a first polypeptide encoded by enough of a protein sequence nucleic acid sequence to retain its biological activity, this first polypeptide is then joined to a the coding sequence for a second polypeptide also containing enough of a polypeptide sequence of a protein to retain its physiological activity. The coding sequence being operatively linked to a control sequence which enables the coding sequence to be expressed in the milk of a transgenic non-human placental mammal.

A DNA sequence which is suitable for directing production to the milk of transgenic animals carries a 5'-promoter region derived from a naturally-derived milk protein and is consequently under the control of hormonal and tissue-specific factors. Such a promoter should therefore be most active in lactating mammary tissue. According to the current invention the promoter so utilized were followed by a DNA sequence directing the production of a protein leader sequence which would direct the secretion of the transgenic protein across the mammary epithelium into the milk. At the other end of the transgenic protein constructs a suitable 3'-sequence, preferably also derived from a naturally secreted milk protein, and may be added to improve stability of mRNA. An example of suitable control sequences for the production of proteins in the milk of transgenic animals are those from the caprine beta casein promoter.

The production of transgenic animals can now be performed using a variety of methods. The method preferred by the current invention is nuclear transfer.

### Therapeutic Uses.

The antibody preparations provided herein is preferably employed for in *vivo* applications. Depending on the intended mode of administration *in vivo* the compositions used may be in the dosage form of solid, semi-solid or liquid such as, e.g., tablets, pills, powders, capsules, gels, ointments, liquids, suspensions, or the like. Preferably the antibody compositions are administered in unit dosage forms suitable for single administration of precise dosage amounts. The compositions may also include, depending on the formulation desired, pharmaceutically acceptable carriers or diluents, which are defined as aqueous-based vehicles commonly used to formulate pharmaceutical compositions for animal or human administration. The diluent is selected so as not to affect the biological activity of the human recombinant protein of interest. Examples of such diluents are distilled water, physiological saline, Ringer's solution, dextrose solution, and Hank's solution. The same diluents may be used to reconstitute lyophilized a human recombinant protein of interest. In addition, the pharmaceutical composition may also include other medicinal agents, pharmaceutical agents, carriers, adjuvants, nontoxic, non-therapeutic, non-immunogenic stabilizers, etc. Effective amounts of such diluent or carrier were amounts which are effective to obtain a pharmaceutically acceptable formulation in terms of solubility of components, biological activity, etc.

The compositions herein may be administered to human patients via oral, parenteral or topical administrations and otherwise systemic forms for anti-melanoma, anti-lymphoma, anti-leukemia and anti-breast cancer treatment.

### Therapeutic Compositions.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they maybe presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the composition may take the form of tablets or lozenges formulated in conventional manner.

### Treatment Methods.

Therapeutic methods involve administering to a subject in need of treatment a therapeutically effective amount of a transgenic antibody. "Therapeutically effective" is employed here to denote the amount of transgenic antibodies that are of sufficient quantity to inhibit or reverse a disease condition (e.g., reduce or inhibit cancer growth). Some methods contemplate combination therapy with known cancer medicaments or therapies, for example, chemotherapy (preferably using compounds of the sort listed above) or radiation. The patient may be a human or non-human animal. A patient typically were in need of treatment when suffering from a cancer characterized by increased levels of receptors that promote cancer maintenance or proliferation.

Administration during in *vivo* treatment may be by any number of routes, including parenteral and oral, but preferably parenteral. Intracapsular, intravenous, intrathecal, and intraperitoneal routes of administration may be employed, generally intravenous is preferred. The skilled artisan did recognize that the route of administration did vary depending on the disorder to be treated.

Determining a therapeutically effective amount specifically did depend on such factors as toxicity and efficacy of the medicament. Toxicity may be determined using methods well known in the art and found in the foregoing references. Efficacy may be determined utilizing the same guidance in conjunction with the methods described below in the Examples. A pharmaceutically effective amount, therefore, is an amount that is deemed by the clinician to be toxicologically tolerable, yet efficacious. Efficacy, for example, were measured by the induction or substantial induction of T lymphocyte cytotoxicity at the targeted tissue or a decrease in mass of the targeted tissue. Suitable dosages were from about 1 mg/kg to 10 mg/kg.

The foregoing is not intended to have identified all of the aspects or embodiments of the invention nor in any way to limit the invention. The accompanying drawings, which are incorporated and constitute part of the specification, illustrate embodiments of the invention, and together with the description, serve to explain the principles of the invention.

All publications and patent applications mentioned in this specification are herein incorporated by reference to the same extent as if each independent publication or patent application is specifically indicated to be incorporated by reference.

While the invention has been described in connection with specific embodiments thereof, it were understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features set forth herein.

### Plasmids

Plasmids utilized:
1) BC2083
2) p80 BC2083 zeo
3) p83 BC2083 DraIII IgG1
4) p96 pCR-BluntII-mayo-heavy
5) p100 BC2083 mayo heavy (BC2197)
6) BC1060
7) p85 pCR-blunt-1060 kappa constant rev
8) p85 pCR-blunt-1060 kappa constant
9) p92 pCR2.1-Blunt-Mayo kappa variable
10) p94 pCR-BluntII-mayo-kap-chim
11) p104 BC1060 mayo LC chim (BC2198)
12) p106 & p107 pCEP4-Mayo-LC (BC2203)
13) p110 pCEP4-BamHl-HC (BC2202)
14) p111 pCR2.1-Mayo-IgGl-heavy-mut
15) p112 pCEP4-Xho-mayo-IgG1-aglycos (BC2206)

### Amino Terminal Sequencing Of The Protein

A murine anti-human CD137 mAb that specifically recognizes human CD137 and does not cross-react with murine CD137. The leading candidate reagent, designated Clone GW, binds specifically to transfected Chinese hamster ovary (CHO) cells expressing human CD137 (CHO/CD137) at levels comparable with commercially available anti-human CD137. Well established methods for the genetic engineering of antibody proteins were used to clone and sequence the anti-CD137 antibody gene variable regions (Maynard and Georgiou 2000; Sacchi, Federico et al. 2001). To identify the family of the antibody, it was sequenced chemically from the amino terminus. In this manner we would be able to use family specific primers for PCR. A purified anti-human 4-1BB antibody was developed from hybridoma GW. The antibody concentration was 670 ng/µl. After reduction in SDS sample buffer, 4.7 µg was run on a 10% Bis-Tris gel in MOPS buffer. After running, the gel was equilibrated in 10 mM CAPS, pH 11.0-10% Methanol buffer and blotted at 100V for 1hr onto Invitrogen PVDF membrane, 0.2 µm pore size (Cat. # LC2002). After brief staining with Coomassie R-250, the bands were submitted for chemical amino terminal sequencing by Edman degradation.

The amino terminal sequencing results for the light chain was DIVLTQSPASLAVSL. This matches MUSIGKM195 or Swissprot KV3B_MOUSE, a member of family 21, a family which is used in about 7% of all antibodies. The amino terminal sequence of the heavy chain is: KVQLQQSGAGLVKPG. This matches MUSIGAPCJ in the Genbank database, a member of family 1, the J558 family which contains the bulk of the germline genes and is used about 30% of the time.

### Sequencing of mRna from Hybridoma

RNA was prepared with a Qiagen RNeasy Mini kit (Cat # 74104). On the 4th day, 13 ml of culture was centrifuged for 5 minutes and resuspended in PBS. It was centrifuged again for 5 min. The pellet was resuspended in 600 µl of RNeasy RLT containing 6 µl of µ-ME. The lysate was passed through a 22g needle 5 times and 600 µl of 70 % EtOH was added and mixed. Seven hundred ul aliquots were applied to the RNeasy column twice, centrifuged 30 seconds, and washed with 700 µl of RWI. It was washed twice with 500 ul of PPE, dried 1 minute, and eluted with 50 µl of water twice.

Two µl of the RNA was reverse transcribed with the Promega Reverse Transcription System (Cat # A3500) using oligodT primer. The reaction was incubated at 42°C for 1 hour and then heated to 95°C for 5 minutes. The reaction was then diluted to 100 µl with water. PCR was carried out on 1 µl aliquots of cDNA using primers chosen from one for the N terminus or 5' end and one for the C terminus or 3' end. PCR was carried out using primers only from the constant region as a positive control. (Figure 1)

PCR products were purified with the Qiagen QiaQuick PCR Purification Kit (Cat #28104). An additional elution was done to make the final volume 100 µl. The absorbance at 260 nm was measured. Concentrations varied from 10-26 ng/µl and 100 ng was given in for sequencing along with the N terminus primer used for the PCR. The sequences obtained are listed in List 1.

Since the amino terminal primers used were part of the coding sequence of the amino terminus of the antibody, they could introduce mutations into the sequence. Using the sequences obtained, the germline genes were identified from the mouse genome. Then primers were synthesized to termini of these genes for the PCR of the entire coding sequence from the cDNA. In this way the entire coding region of the antibody were obtained free of any sequences contributed by PCR primers. The coding sequence is the sequence of the expressed antibody since it is consistent with the amino terminus sequence in each case. The J regions were identified from the known J regions as in annotated in the sequence.

### Variable Regions Chimerized to Human IgG1

One problem with using mouse monoclonal antibodies for therapy is the development of human anti-mouse antibodies (HAMA). Once this occurs, the patient antibodies attack the MAbs, resulting in MAbs complexation, which reduces the circulating time ofMAbs and their binding to target tumor, thereby limiting the antibody anticancer activity. HAMA may also cause allergic reactions upon second exposure to the antibody, and these reactions range from the uncomfortable to the potentially life threatening. Even monoclonal antibodies derived partly or entirely from human cell lines can evoke antibody responses. Chimeric antibodies can elicit human antichimeric antibody (HACA) responses and even human MAbs can evoke human antihuman antibody (HAHA) responses. Commonly, MAbs are partly 'humanized' through genetic engineering. The most common method of antibody humanization involves replacement of the constant region of the mouse MAb with a human constant region, resulting in a mouse:human chimera. Chimeric antibodies are created by cloning the murine gene that codes for the antibody variable region and the human gene that codes for the antibody constant region. This type of genetic engineering enables scientists to produce antibodies with a murine variable region combined with a human constant region. Potential advantages for chimeric antibodies include less immunogenicity and longer circulation of the antibody (LoBuglio, Wheeler et al. 1989; Knight, Wagner et al. 1995). An antibody which stimulates 4-1BB has been reported to suppress antigen-induced humoral immune response (Hong, Lee et al. 2000).

### Construction Of Heavy Chain Chimera And Insertion Into Expression Vector

In order to construct the heavy chain chimera whereby the mouse IgG2a constant region is replaced by the human IgGl constant region, the BC2083 expression vector containing the Immunogen human antibody sequences with a mouse leader sequence was used (Plasmid 1). This gene has a splice donor site eliminated by a G to A silent mutation which did not change the coding for glycine near the C terminus. Unique sites were put into the BC2083 expression vector surrounding the variable region. DraIII and PmlI were put into the N terminus and ApaI exists in the amino portion of the heavy constant region. These sites were cloned into the XhoI sites of BC2083 by PCR of the zeo gene from CMV-Zeo with the primers to give p80 BC2083 zeo (Plasmid 2). This rapid method of restriction site insertion into plasmids utilizes the zeocin resistance conferred by the zeo gene. Zeocin resistance is selected for by using 25 ug/ml of zeocin in NZYCM agar.

The human IgG1 constant portion was put back into the unique ApaI and XhoI sites by cutting it out of BC2083 and cloning it into p80 to give p83 BC2083 DraIII IgGl (Plasmid 3). This plasmid has unique DraIII/Pm1I and ApaI sites flanking the heavy variable region so that any heavy variable region were attached to the human IgGl constant region coding sequences.

The heavy chain variable region of the anti-4-1BB antibody was prepared for insertion by putting DraIII and Pm1I sites on the amino terminus and an Apa site on the C terminus by PCR. The ApaI site is naturally occurring near the amino terminus of the human IgG1 constant region. PCR was performed with primers MHE and MHECusing PfuTurbo (Stratagene Cat No. 600153-81) and cDNA. The PCR fragment was cloned into pCR-BluntII- TOPO (Invitrogen Cat. No.: K28602) and sequenced with primers pcr2.1 f and pcr2.1b (List 3). This give p96, containing the heavy chain variable region flanked by DraIII-PmII and ApaI (Plasmid 4).

The beta-casein expression vector, p100 BC2083 mayo heavy (BC2197)(Plasmid 5), was constructed by isolating the p96 pCR-BluntII-mayo-heavy DraIII-Apal fragment and ligating it to cut DraIII- ApaI cut p83 BC2083 DraIII IgG1 (Plasmid 3).

### Construction Of Light Chain Chimera And Insertion Into Expression Vector

The expression vector used for the light chain was BC1060 (Plasmid 6). To enable the fusion of the variable region to the human kappa constant region, two restriction sites were engineered into the mouse J region in order. A KpnI site was introduced by changing the codon for a glycine from GGG or GGC to GGT. The coding sequence for a leucine was changed to CTT from CTG to create a HindIII site (plasmid 8).

Using PCR with PfuTurbo (Stratagene) the coding region of the human constant region of the kappa chain was isolated from BC 1060 with KpnI and HindIII sites at the beginning and a naturally occurring SacI site near the end of the coding region using primers from Table 9. This PCR product was cloned into ZERO Blunt TOPO PCR W EC (Invitrogen Cat. No.: K286020). These plasmids were sequenced and the resulting sequences are listed in List 4. This makes p85 pcr-blunt-1060 kappa constant rev (Plasmid 7) and p86 per-blunt -1060 kappa constant (Plasmid 8).

Similarly, the variable region was isolated from cDNA by PCR with primers from Table 10 and cloned into pCR2.1-Blunt-TOPO to make p92 pCR2.1-Blunt-Mayo kappa variable (Plasmid 9) where the variable region is flanked by a XhoI site at nucleotide 340 and KpnI and HindIIII sites around nucleotide 731. These plasmids were sequenced and the resulting sequences are listed in List 5.

The light chain chimera was first constructed in pCR-Blunt using 3 pieces of DNA. The backbone from XhoI to SacI was contributed by p86 pcr-blunt-1060 kappa constant. The kappa constant region was the HindIII-SacI piece from p85 pcr-blunt-1060 kappa constant rev. The variable region was supplied by p92 pCR-Blunt-Mayo kappa variable rev using the XhoI-HindIII piece. Colonies were checked by PCR with primers, pcr2.1f and pcr2.1b, looking for production of a 863 bp fragment. This gives p94 pCR-BluntII-mayo-kap-chim (Plasmid 10). The plasmid was checked by cutting with XhoI and SacI to give a 684 bp fragment.

The light chain chimera was put into the beta-casein expression vector BC1060 containing the Immunogen human light chain with the mouse heavy leader sequence. p94 was cut with XhoI-SacI and the small piece isolated. BC1060 was cut with KpnI-SacI and the 5206 bp piece isolated. BC1060 was cut with KpnI, XhoI, and PacI to isolate the large backbone. These three pieces were ligated and colonies were screened with the needed primers. The positive plasmid was checked with BgIII and the PCR product sequenced. This plasmid is p104 BC1060 mayo LC chim (BC2198)(Plasxnid 11).

### Construction Of Cell Vulture Expression Vectors

The recent large-scale transient transfection technology is now generating great interest because of its demonstrated ability to produce large amounts of recombinant proteins within a few days. The human embryonic kidney 293 cell line (293) is suitable for transient transfection technology as it were efficiently transfected. Moreover, a 293 genetic variant stably expressing the EBV EBNA1 protein (293E) has been shown to provide significantly higher protein expression when EBV's oriP is present in the vector backbone. The increased expression obtained by the use of oriP/EBNA1 systems appears to be independent of episomal replication when performing transient transfection. This is supported by the fact that removal of the DS domain of oriP, which is responsible for initiation of DNA replication in EBNA1 positive cells does not reduce transgene expression, while removal of FR but not DS strongly reduces expression. The increased expression is thus likely due to the combined effect of the EBNA1- dependent enhancer activity of oriP and to the increased nuclear import of plasmids owing to the presence of a nuclear localization signal in EBNA1. (Pham et al., 2003) pCEP4 (Invitrogen, Cat # V04450) a vector designed for high-level, constitutive expression from the CMV promoter. The vector contains the EBNA-1 gene for episomal expression in primate cell lines. The utility of the pCEP4 vector has been found to be limited to the human 293 EBNA cell line (Parham et al., 2001). The 293EBNA/ebv vector host system represents a significant improvement over COS7/SV40ori based systems. (Jalanko et al., 1988; Shen et al., 1995) An important issue for high level recombinant protein expression is to use vectors with promoters that are highly active in the host cell line, such as the CMV promoter, which is particularly powerful in 293 cells where it has been shown to be strongly transactivated by the constitutively expressed adenovirus E1a protein. (Durocher et al., 2002).

To construct the transient expression vector for the light chain, the XhoI fragment from p104 BC1060 Mayo LC chim (Plasmid 11) was ligated into the XhoI site of pCEP4 to give p106 and p107 pCEP4-Mayo-LC (#2203) (Plasmid 12). Positive colonies were detected by PCR with oligos CEPF & KVC.

To construct the transient expression vector for the heavy chain, the BamHl fragment of p100 BC2083 Mayo heavy was cloned into BamHI cut pCEP4. Colonies were screened by PCR with HVC 09 and CEPF. This resulted in plasmid p110 pCEP4-BamHI-HC (#2202)(Plasmid 13)

### Mutagenesis of Glycosylation Site in IgG1.

Antibodies are glycosylated at Asn297 of the heavy chain constant region (Wright and Morrison, 1998). The carbohydrate is sequestered between the heavy chains and has a complex biantennary structure composed of a core saccharide structure consisting of two mannosyl residues attached to a mannosyl-di-N-acetylohitobiose unit (Rademacher et al. 1985). The outer arms arise from the terminal processing of the oligosaccharide in the Golgi; although the overall structure of the carbohydrate is conserved, considerable heterogeneity is seen in the identity of the terminal sugar residues. Analysis of carbohydrates isolated from normal human serum IgG has yielded up to 30 different structures. Somewhat fewer structures have been enumerated in other mammals (Mizuochi et al. 1982), but the biantennary structure is conserved. One important aspect of purifying recombinant proteins from any expression system is demonstrating that the final product has a glycosylation pattern that is comparable to the native protein, but this is difficult given the natural micro-heterogeneity in carbohydrate structures. Failure to achieve comparable glycosylation during protein expression could lead to the addition of specific carbohydrate processing steps during purification, which would add complexity and cost Having an aglycosylated IgG product that lacked carbohydrates would simplify purification and allow us to develop a more efficient and consistent high-yield process to produce clinical-grade preparations. Antibodies lacking glycosylation lack effector functions like antibody mediated cell dependent cytotoxicity (ADCC) since they can not bind Fc gammaR1 receptor and complement activation by their failure to bind Clq (Nose and Wigzell 1983; Leatherbarrow et al. 1985; Tao 1989; Jefferis et al. 1998; Mimura et al. 2000; Mimura et al. 2001; Dorai et al. 1991).. They can still bind the neonatal receptor. (Simmons et al. 2002) Since the Mayo anti-4-1BB antibody is an agonist antibody and, like 4-1BB ligand, activates the 4-1BB receptor loss of effector functions is not detrimental and would possibly be beneficial.

### Glycosylation Variants

The oligosaccharides from all transgenic animals (goats & mice) were a mixture of high mannose, hybrid and complex type oligosaccharides with or without fucose. Sialic acid was present as 2, 6-linked sialic acid and no a 1,3-linked galactose was observed in the transgenic glycoprotein. These results indicate that transgenic animals with closely related genetic backgrounds express recombinant protein with comparable glycosylation. The absence of CH2-associated carbohydrate is thought to cause conformational changes in the CH2 and hinge regions that are unfavorable to the interaction with effector molecules and thus result in loss of function. According to the current invention certain alterations in carbohydrate structure also can affect antibody function or therapeutic effectiveness. In diseases such as rheumatoid arthritis, a higher than normal incidence of agalactosyl structures (which seems to be specific for IgG Fc-associated carbohydrate) has been documented (Parekh et al. 1985; Rademacher et al. 1988a). It has been proposed that this aglycosylated structure is more mobile than the structure normally seen in this region and thus may induce changes in the quaternary structure of the glycoprotein, contribute to the immunogenicity of the Ab, or may itself contribute to aberrant antibody function (Rademacher et al. 1988b; Axford et al. 1992). In the disease state, however, this structure is only one of numerous glycoforms observed.

To block the glycosylation by changing the target asparagine to a glutamine, the heavy chain coding sequence was prepared by PCR with PfuTurbo from BC2083 using primers heavy constant N and heavy constant C subcloned into pCR-Zero -Blunt. This gave p76 and p77 pCR2.1-Blunt-IgGl-heavy-constant. These plasmids were sequenced giving the sequences in List 6 to ensure no mutations were introduced into the constant region during the PCR.

According to the current invention the 4-1BB antibodies produced by transgenic mice and goats augment the initial graft versus host disease and stimulates the EMH. This then requires Fc cross-linking which may explain differences between "g" and "gw". Animals that die in the experiments provided in the development of the current invention likely die of GVH secondary to a cytokine cascade. Also according to the current invention, the aglycosylated antibodies developed stimulate 4-1BB and result in prolonged survival in the whole animal lymphoma model. Therefore, according to the current invention the aglycosylated antibodies (chimeric humanized and human) have beneficial attributes for the treatment of cancer and autoimmune disorders. This while the glycosylated version has treatment potential for BMT conditions and those of similar cause.

### Construction of Clones

The subcloned constant region in p77 was mutagenized using the QuickChange XL Mutagenesis (Stratagene) kit and the mutagenic oligos. This oligo changes asparagine 297 to a glutamine and removes a nearby BsaAI site to facilitate screening by restriction enzyme analysis by the silent mutation of a threonine codon. This gave plasmids p88, p89 and p90 pCR2.1 -Blunt-IgGl-heavy-mut. PCR was carried out on these plasmids with the primers to prepare a fragment for sequencing to give the sequences in List 7

The chimera with the heavy chain variable region of the anti-CD137 antibody was prepared by ligating the small KpnI-AgeI piece of#110 pCEP4-BamHI-HC (#2202) containing the variable region into KpnI-AgeI cut #88 pCR2.1-Blunt-IgG1-heavy-mut. This gives plasmid p111 pCR2.1-Mayo-IgGl-heavy-mut (Plasmid 14). This plasmid was checked with BsaAI-PstI.

To construct the transient expression vector, the small XhoI fragment from p111 containing the chimeric antibody coding region was inserted into the XhoI site of pCEP4. Colonies were checked by PCR with HVC C09 and CEPF. This gave p112 pCEP4-Xho-mayo-IgG1-aglycos (BC2206). Expected fragments were obtained with EcoRV- HindIII digestion (2479 bp) and BamHI digestion (1454 bp).

To construct the beta casein expression vector, the small XhoI fragment from p111 containing the chimeric antibody coding region was inserted into the XhoI site of BC2083. Colonies were checked by PCR with oligos HVC 09 and CAS. Digestion with MluI-Bco47III-NotI gave the expected 2479 bp fragment, while digestion with BamHI gave the expected 1454 bp fragment.

### Expression of Aglycosylated Anti-CD137

293 cells were transfected. Running a 12% gel did not give enough separation from the serum proteins and interfered with the detection of the heavy chain. A 4-12% gradient gel gave good enough separation.

### Antibody Humanization

It has been established that stimulation of CD 137 through its natural ligand or agonistic antibodies potentiates the antitumor immune response *in vivo* through stimulation of tumor-reactive effector T-cells and enhanced regulatory NK activity. Systemic administration of anti-murine CD137 monoclonal antibodies (mAbs) induced complete regression of large tumors in mice such as the poorly immunogenic AGF104A sarcoma and the highly tumorigenic P815 mastocytoma, as well as EL*A* thymoma, K1735 melanoma, B10.2 and 87 sarcoma, RENCA renal carcinoma, J558 plasmacytoma, MCA205 sarcoma, JC breast cancer, MCA26 colon cancer and GL261 glioma, alone or in combination with other therapeutic modalities. Several poorly immunogenic tumors required prior priming of the T-cell response by immunization with tumor-derived peptide, which suggests that combination therapy may increase the efficacy of anti-CD137 *in vivo.* CD137 agonistic antibodies elicit potent T-cell responses but their role in humoral immune responses is inhibitory. Systemic administration of anti-mouse CD137 mAb suppresses antigen-specific antibody production by energizing T-helper cells and inhibits autoantibody production by deleting autoreactive B cells. This unique feature of CD137 signaling has important clinical implications because it may minimize the Human Anti-Mouse Antibody (HAMA) response, which inactivates murine antibody proteins in the circulation.

Extensive studies demonstrated that stimulation of CD137 by its natural ligand or by agonistic antibodies potentiated an anti-tumor response that resulted in regression of established mouse tumors in various models. Anti-CD137 offers great promise as a potential therapeutic agent against certain solid tumors. The difficulties not addressed by the prior art include the development of an anti-CD137 for human cancer therapeutic suitable for clinical applications, that is, to demonstrate its effect against human tumors and to establish a reliable and cost-effective production source. Specifically, a chimeric or humanized agonistic anti-CD137 antibody that: 1) contains human constant region sequences on the heavy and light chains of the immunoglobulin molecule (IgG) to minimize neutralization by Human Anti-Mouse Antibody (HAMA) responses in vivo; and 2) lacks the glycosyl group found on native IgG to simplify antibody purification from the milk of transgenic goats. The above would also be true of a fully human anti CD137 antibody.

Humanization (also called Reshaping or CDR grafting) is an established technique for reducing the immunogenicity of monoclonal antibodies (mAbs) from xenogeneic sources, such as mice in the current invention, and improving the activation of the altered antibody in the human immune system.

Although the mechanics of producing the engineered mAb using the techniques of molecular biology are relatively straightforward, simple grafting of the rodent complementarity-determining regions (CDRs) into human frameworks may not always reconstitute the binding affinity and specificity of the original mAb. In order to humanize an antibody, as with the current invention, the critical step in reproducing the function of the original molecule and design choices along that path. These design elements include choices: the extents of the CDRs, the human frameworks to use and the substitution of residues from the rodent mAb into the human framework regions (back mutations). The positions of the back mutations, if any, are identified principally by sequence/structural analysis or by analysis of a homology model of the variable regions' 3D structure.

According to the current invention, a mouse-human chimeric monoclonal antibody agonist anti-CD137, was developed. Humanization of the anti-CD137 antibody is expected to enhance its use for patients undergoing immunotherapy or for other indications. On the basis of the observed amino acid sequence identity, complementary determining regions (CDRs) of the VL and VH regions were grafted onto the human anti-DNA-associated idiotype immunoglobulin clone. It was observed by competitive ELISA that the recombinant chimeric antibody of the invention exhibited a similar bioactivity profile when compared with the murine monoclonal antibody. The anti-CD137 antibody was effective in mediating both antibody-dependent cellular cytotoxicity and complement-mediated cytotoxicity when assayed. Humanization of the antibody sequences of the current invention are expected to eliminate any undesired human anti-mouse antibody response, allowing for repeated i.v. administration into humans.

### Comparison of Anti-CD 137 Antibody Carbohydrates

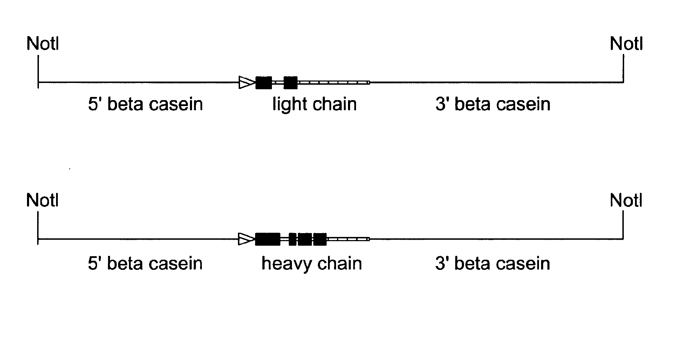

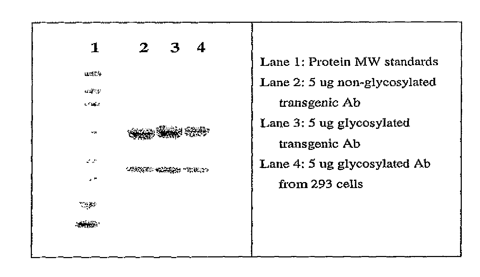

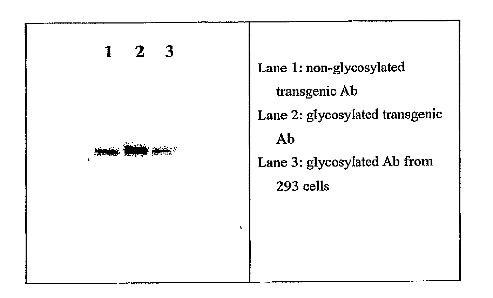

The anti CD137 expressed from transgenic animal and human 293 cells were compared. SDS-PAGE of both glycosylated antibodies shows similar pattern while the heavy chain from non-glycosylated antibody migrated slightly faster. However, all these three antibodies were recognized by an anti human IgG (Fc specific) on western blot. Results from MALDI-TOF analysis show different oligosaccharides present in glycosylated antibodies from the different expression systems. The major oligosaccharide in transgenic antibody is Man5 without fucose and minor species are G1F, G2F, Man6 and G1. However, anti CD137 antibody from human 293 cell line contains mainly fucosylated oligosaccharides including G0F and G1F. G2F is also present as minor species. The binding of transgenic glycosylated and non-glycosylated anti CD137 to lectin columns was also investigated. It was found that the majority of transgenic glycosylated antibody bound to Con A, a lectin specific for high mannose type carbohydrates. The interaction between antibody and Con A confirms the presence of high mannose type oligosaccharides present on transgenic glycosylated antibody. See Figures 21-25. It is also possible that increasing the ADCC levels could enhance the effectiveness of anti-CD-137 antibodies. This could be done by any number of methods.

Turning to Figure 21, the results indicates that glycosylated anti CD137 antibodies from either transgenic animal or human 293 cell line migrated in similar pattern. As expected, the heavy chain from non-glycosylated transgenic antibody migrated slightly faster, indicating the absence of carbohydrates. However, the staining intensities of these antibodies in the same quantity were slightly different on the gel. The difference between glycosylated antibodies from transgenic animal and human 293 cell line may result from different protein quantitation assays.

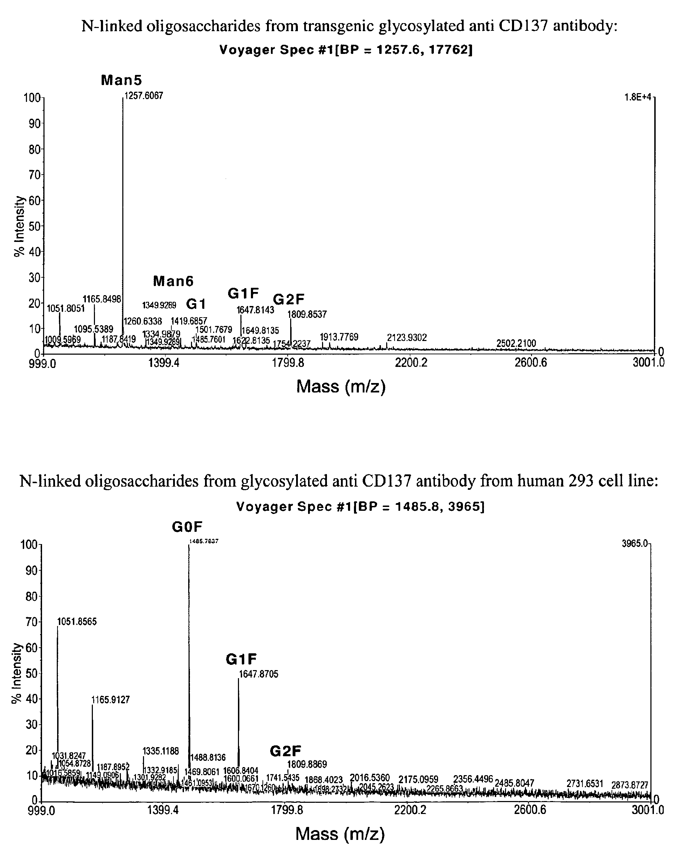

The three antibodies in 0.5 ug were also applied to a 4-20% SDS-PAGE and transferred to a PVDF membrane. A western blot was performed using a goat anti human IgG (Fc specific) antibody. The result is shown in figure 22..

Turning to Figure 22, as expected, all three antibodies were recognized by anti human IgG (Fc specific) antibody because they are humanized forms.

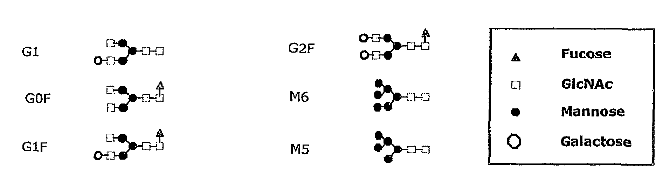

The carbohydrates were released using PNGase F in the presence of 1% β-mercaptoethanol from glycosylated antibodies. MALDI-TOF analysis was performed. The result is shown in Figures 23(a)-(c).

Turning to Figures 23(a)-(c), the carbohydrate profiles are identified in the transgenic antibody vis-à-vis. The major carbohydrate in transgenic antibody is non-fucosylated Man5. There are some minor carbohydrate species including core fucose containing oligosaccharides (G1F and G2F) and non-fucosylated oligosaccharides (G1 and Man6). However, the carbohydrates identified in the same antibody expressed from human 293 cell line are mostly fucosylated oligosaccharides. The major structures in these oligosaccharides are G0F and G1F. There is also G2F as minor species.

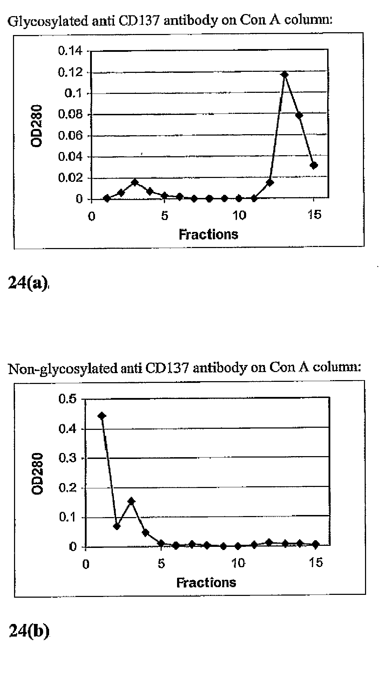

Turning to Figures 24(a)-(b), the lectins were also used to confirm the presence of specific carbohydrates in transgenic antibody. The following figures show the chromatographes of glycosylated and non-glycosylated transgenic antibodies on Con A column. The results from Figures 24(a)-(b) show that the majority of glycosylated transgenic antibody bound to Con A column and eluted by α-methylmannoside starting from fraction 11. In contrast, most of non-glycosylated anti CD137 antibody and an antibody without any high mannose oligosaccharides (data not shown) were not bound. The data is consistent with the MALDI-TOF analysis and indicate that the presence of high mannose type oligosaccharides in transgenic glycosylated antibody.

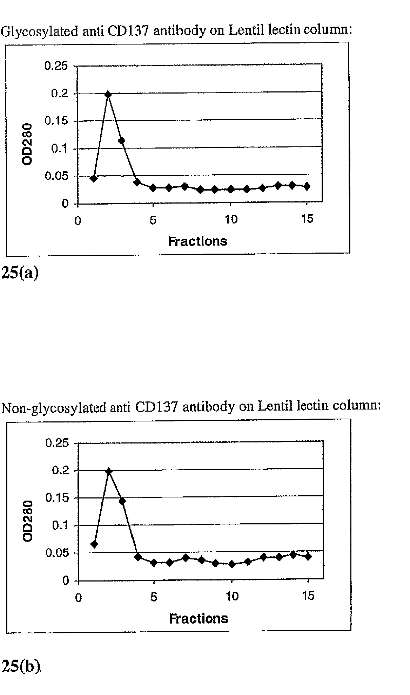

Turning to Figures 25(a)-(b), the Lentil lectin column was also used to determine the presence of core fucose because the lectin is known to interact with core fucosylated oligosaccharides. Both glycosylated and non-glyeosylated transgenic antibodies were applied to a Lentil lectin column, respectively. The bound protein was eluted by α-methylmannoside. It was found that neither of these antibodies bound to the lectin column. Very surprisingly, an antibody, which contains mainly core fucosylated oligosaccharides, also didn't bind to the column (data not shown). However, Majority of a control glycoprotein bound to the column (data not shown). The result suggests that the core fucose in some of the antibody may not expose or be accessible to the lectin column. Therefore, the binding of antibody to Lentil lectin column cannot be used as tool to determine the presence of core fucose in the antibody studied.

### Cloning IgG1 mutant

The Mayo anti-CD137 antibody was previously expressed in mouse milk. For mouse expression, the construction of BC2197 (p100 BC2083 mayo heavy) and (BC2198) p104 BC1060 mayo LC chim were described in the quarterly report of September 2003. The parental plasmids were those of the Immunogen antibody expression vectors, BC2083 for the heavy chain and BC 1060 for the light chain. Basically, the variable regions including the leader sequences in those parental plasmids were exchanged with the cDNA sequence from the variable region of the heavy and light chains of the Mayo anti-CD137 antibody cDNA. In this report, for the goat expression vector, we replaced the constant regions, IgG1 of the heavy chain and kappa of the light chain with sequences which were cloned at GTC.

### Cloning of IgG1 sequences

The heavy chain was cloned from cDNA purchased from Invitrogen. PCR with PfuTurbo was performed using placental cDNA and the primers shown in figure 1. The C terminus primer 61960C11 has a base change with respect to the wild type sequence to destroy a splice donor site. The 993 bp fragment was cloned into ZeroBlunt. The sequences, indicated that one sequence was of the Glm(3). There are inherited differences associated with Gamma-globulin of human serum (Grubb 1956; Grubb and Laurell 1956). There is a similar system for Km (Kappa marker, previously referred as Inv (or Inv) which stands for ëInhibitrice Virmi). This is the Caucasian allotype Glm(f) or G1m(3) instead of the African allotype G1m(z) or Glum(17) as found in Immunogen. (Initially the immunoglobulin phenotypes were described by alphabetical notations. However, the growing complexity of these system, clashes of notation and doubts as to synonymy lead to the holding of a WHO sponsored conference. The committee of World Health Organization (WHO 1964, 1976) recommended numerical notation for the antigenic types of these systems.) In fact all of the antibodies we have produced have the Glm(17) allotype except for BR96 which has Glm(3). IgG3 and IgG4 have arg in this position. Another allotype is G1m(1) or Glm(a) which is Arg-Asp-Glu-Leu in positions 355-358 (EU numbering for complete chain). In the non 1 or non-a allele the sequence is Arg-Glu-Glu-Met. Only Neuralab of our produced antibodies has the Glm(non-1) marker.

In order to clone the other allotype, PCR was done from brain cDNA as above to give plasmids, p116, p117, p118, and p119. None of these plasmids had the correct sequence. For example, most of the plasmids were missing the ApaI and/or the XhoI sites at the end of the sequence, which should have been provided by the PCR primers. The PCR was done again using p 116 as template or brain cDNA again.

PCR of p 116 yielded 121, 122, and 123. PCR of brain cDNA yielded 124. The insert from plasmid 121 was used to make p133, p134, p135, and p136 which are BC2083 mayo heavy G1m(17) by cutting 100c BC2083 mayo heavy with ApaI & XhoI and p121 ZeroBlunt-IgG1 Glm(17) with ApaI & XhoI, ligating and selecting on kanamycin. p133 was used.

For the mouse expression, only the variable regions was changed in plasmid BC2083, an expression vector containing the human antibody sequences with a mouse leader sequence was used. This gene has a splice donor site at the end of the IgG1 constant region eliminated by a G to A silent mutation which did not change the coding for glycine. For the goat expression, the constant region was changed to an IgG1. constant region that was cloned. The cloned constant region from p114 was used to create p137 and 138. p100 BC2083 mayo heavy (BC2197) was cut with ApaI -XhoI and 114 ZeroBlunt-IgGI G1m(3) cut with ApaI & XhoI and ligated to give p138 (BC2228). The cloned constant region from p121 (Glm(17)) was used to create p133, 134,135, and 136 BC2083 mayo heavy Glum(17). The kappa constant region was also replaced with one cloned at GTC. (May 11, 2004)

### SEQ. ID. NO.

primer 1 (diluted 4 ul + 4 ul H2O) 5'
AGGGTACCAAGCTTGAAATCAAACGAAC Kappa Constant Human H01 1

### SEQ. ID. NO.2

primer 2 (diluted 1 ul + 7 ul H2O) 5'
AAGGGTCCGGATCCTCGAGGATCCTAACACTCTCCCCTGTTGAAGCTC Human Kappa C #7734

This PCR product was rePCRed with the same primers and cloned into the Invitrogen plasmid ZeroBlunt to give plasmids 127, 128, 129, and 130.

### Literature Cited and incorporated by Reference:

1. Axford, J. S., N. Sumar, et al. (1992), Changes in normal glycosylation mechanisms in autoimmune rheumatic disease, J. CLIN INVEST 89(3): 1021-31.
2. Baguisi A, et al. (1999), Production of Goats by Somatic Cell Nuclear Transfer, NATURE BIOTECH, 17: 456-461.
3. Blake J, Johnston JV, Hellström KE, Marquardt H & Chen L. (1996), Use of combinatorial peptide libraries to construct tumor epitopes recognized by MHC class I-restricted cytolytic T lymphocytes, J. EXP. MED. 184:121-30.
4. Boyd, P. N., A. C. Lines, et al. (1995), The effect of the removal of sialic acid, galactose and total carbohydrate on the functional activity of Campath-1H, Mol. Immunol. 32(17-18): 1311-8.
5. CanBeld, S. M. and S. L. Morrison (1991) The binding affinity of human IgG for its high affinity Fc receptor is determined by multiple amino acids in the CH2 domain and is modulated by the hinge region, J. EXP. MED. 173(6): 1483-91.
6. Chen, S.H., et al. (2000), Rejection of disseminated metastases of colon carcinoma by synergism of IL-12 gene therapy and 4-IBB costimulation, MOL. THER., 2:39-46.
7. Chiu M.H. et al. (1994), In vivo targeting function of N-linked oligosaccharides with terminating galactose and N-acetylgalactosamine residues, J. BIOL. CHEM., 269 (23):16195-202).
8. Dorai, H., B. M. Mueller, et al. (1991), Aglycosylated chimeric mouse/human IgGl antibody retains some effector function, HYBRIDOMA 10(2): 211-7.
9. Foell, J. et al. (2003), CD137 costimulatory T cell receptor engagement reverses acute disease in lupus-prone NZB x NZW FT mice, J. CLIN. INVEST. 111 (10), 1505-1518..
10. Fujii, S., T. Nishiura, et al. (1990), Structural heterogeneity of sugar chains in immunoglobulin G. Conformation of immunoglobulin G molecule and substrate specificities of glycosyltransferases, J. BIOL, CHEM. 265(11): 6009-18.
11. Guinn, B.A. et al. (1999), 4-1BB Cooperates With B7-1 And B7-2 In Converting A B Cell Lymphoma Cell Line Into A Long-Lasting Antitumor-Vaccine, J. IMMUNOL. 162:5003-5010.
12. Hong, H. J., J. W. Lee, et al. (2000), A humanized anti--4-1BB monoclonal antibody suppresses antigen-induced humoral immune response in nonhuman primates, J. IMMUNOTHER. 23(6): 613-21.
13. Ip, C. C., W. J. Miller, et al. (1994), Structural characterization of the N-glycans of a humanized anti-CD18 murine immunoglobulin G, ARCH BIOCHEM BIOPHYS 308(2): 387-99.
14. Jalanko, A., A. Kallio, et al. (1988), An EBV-based mammalian cell expression vector for efficient expression of cloned coding sequences, BIOCHIM BIOPHYS ACTA 949(2): 206-12.
15. Jefferis, R., J. Lund, et al. (1995), Recognition sites on human IgG for Fc gamma receptors: the role of glycosylation, IMMUNOL LETT 44(2-3): 111-7.
16. Johnston JV, Malacko AR, Mizuno M, McGowan P, Hellström I, Hellström KE, Marquardt H & Chen L. (1996), B7-CD28 costimulation unveils the hierarchy of tumor epitopes recognized by MHC class I-restricted CD8 cytolytic T lymphocytes, J. EXP. MED. 183:791-800
17. Kasinathan P. et al. (2001), Production of Calves from G1 Fibroblasts, NATURE BIOTECH, 19: 1176-1178.
18. Kim et al. (2001), Divergent Effects of 4-1BB Antibodies on Antitumor Immunity and on TumorReactive T-Cell Generation, CANCER RESEARCH 61: 2031-2037.
19. Kim, J. K., M. F. Tsen, et al. (1994), Catabolism of the murine IgG1 molecule: evidence that both CH2-CH3 domain interfaces are required for persistence of IgG1 in the circulation of mice, SCAND. J. IMMUNOL. 40(4): 457-65.
20. Knight, D. M., C. Wagner, et al. (1995), The immunogenicity of the 7E3 murine monoclonal Fab antibody fragment variable region is dramatically reduced in humans by substitution of human for murine constant regions, MOL. IMMUNOL 32(16): 1271-81.
21. Kumpel, B. M., T. W. Rademacher, et al. (1994), Galactosylation of human IgG monoclonal anti-D produced by EBV-transformed B-lymphoblastoid cell lines is dependent on culture method and affects Fc receptor-mediated functional activity, HUM ANTIBODIES HYBRIDOMAS 5(3-4): 143-51.
22. Leatherbarrow, R. J., T. W. Rademacher, et al. (1985) Effector functions of a monoclonal aglycosylated mouse IgG2a: binding and activation of complement component C1 and interaction with human monocyte Fc receptor, MOL. IMMUNOL. 22(4): 407-15.
23. Li, Q., et al. (2003), Polarization Effects of 4-1BB during CD28 Costimulation in Generating Tumor-reactive T Cells for Cancer Immunotherapy, CANCER RESEARCH 63(10): 2546-2552.
24. Li Y, Hellström KE, Newby SA & Chen L. (1996), Costimulation by CD48 and B7-1 induces immunity against poorly immunogenic tumors, J. EXP. MED. 183:639-44.
25. LoBuglio, A. F., R. H. Wheeler, et al. (1989), Mouse/human chimeric monoclonal antibody in man: kinetics and immune response, PROC. NATL. ACAD. SCI. U S A 86(11): 4220-4.
26. Lund, J., N. Takahashi, et al. (1993), Control of IgG/Fc glycosylation: a comparison of oligosaccharides from chimeric human/mouse and mouse subclass immunoglobulin Gs, MOL. IMMUNOL. 30(8): 741-8.
27. Lund, J., N. Takahashi, et al. (1996), Multiple interactions of IgG with its core oligosaccharide can modulate recognition by complement and human Fc gamma receptor I and influence the synthesis of its oligosaccharide chains, J. IMMUNOL. 157(11): 4963-9.
28. Lund, J., N. Takahashi, et al. (1995), Oligosaccharide-protein interactions in IgG can modulate recognition by Fc gamma receptors, FASEB. J. 9(1): 115-9.
29. Malaise, M. G., C. Hoyoux, et al. (1990), Evidence for a role of accessible galactosyl or mannosyl residues of Fc domain in the in vivo clearance of IgG antibody-coated autologous erythrocytes in the rat, CLIN.IMMUNOL. IMMUNOPATHOL. 54(3): 469-83.
30. Malhotra, R., M. R. Wormald, et al. (1995), Glycosylation changes of IgG associated with rheumatoid arthritis can activate complement via the mannose-binding protein, NAT. MED. 1(3): 237-43.
31. Martinet, O., et al. (2000), Immunomodulatory gene therapy with interleukin-12 and 4-1BB ligand: long-term remission of liver metastases in a mouse model, J. NATL. CANCER Inst. 92:931-936.
32. Maynard, J. and G: Georgiou (2000), Antibody engineering, ANNUAL REV. BIOMED. Eng 2: 339-76.
33. Melero, I., et al. (1997), Monoclonal antibodies against the 4-1BB T-cell activation molecule eradicate established tumors, NAT. MED. 3:682-685. BMS.
34. Melero, I., et al. (1998), Amplification of tumor immunity by gene transfer of the co-stimulatory 4-1BB ligand: synergy with the CD28 co-stimulatory pathway, EUR. J. IMMUNOL. 28:1116-1121.
35. Meng L, et a1. (Aug. 1997), Rhesus Monkeys Produced by Nuclear Transfer, BIOLREPROD., 57(2):454-9.
36. Mimura, Y., S. Church, et al. (2000), The influence of glycosylation on the thermal stability and effector function expression of human IgG1-Fc: properties of a series of truncated glycoforms, MOL. IMMUNOL. 37(12-13): G97-706.
37. Mimura, Y., P. Sondermann, et al. (2001), Role of oligosaccharide residues of IgG1-Fc in Fc gamma PIIb binding, J. BIOL. CHEM. 276(49): 45539-47.
38. Miller et al. (2002), 4-1BB-Specific Monoclonal Antibody Promotes the Generation of Tumor-Specific Immune Responses by Direct Activation of CD8 T Cells in a CD40-Dependent Manner, THE JOURNAL OF IMMUNOLOGY, 169: 1792-1800. Immunex.
39. Mittler, R.S., et al. (1999), Anti-4-1BB monoclonal antibodies abrogate T cell-dependent humoral immune responses in vivo through the induction of helper T cell energy, J. EXP . MED. 190:1535.
40. Mizuochi, T., T. Taniguchi, et al. (1982), Structural and numerical variations of the carbohydrate moiety of immunoglobulin G, J. IMMUNOL. 129(5): 2016-20.
41. Nose, M. and H. Wigzell (1983), Biological significance of carbohydrate chains on monoclonal antibodies, Proc. Natl. Acad. Sci. U S A 80(21): 6632-6.
42. Parekh, R. B., R. A. Dwek, et al. (1985), Association of rheumatoid arthritis and primary osteoarthritis with changes in the glycosylation pattern of total serum IgG., NATURE 316(6027): 452-7.
43. Parekh, R. B., A. G. Tse, et a1. (1987), Tissue-specific N-glycosylation, site-specific oligosaccharide patterns and lentil lectin recognition of rat Thy-1, EMBO. J. 6(5): 1233-44.
44. Parham, J. H., T. Kost, et al. (2001), Effects of pCIneo and pCEP4 expression vectors on transient and stable protein production in human and simian cell lines, CYTOTECHNOLOGY 35(3): 181-187.
45. Pham, P. L., S. Perret, et al. (2003), Large-scale transient transfection of serum-free suspension-growing HEK293 EBNA1 cells: Peptone additives improve cell growth and transfection efficiency, BIOTECHNOL BIOENG 84(3): 332-42.
46. Pound, J. D., J. Lund, et al. (1993), Aglycosylated chimaeric human IgG3 can trigger the human phagocyte respiratory burst, MOL. IMMUNOL. 30(3): 233-41.
47. Rademacher, T. W. (1993), Glycosylation as a factor affecting product consistency, BIOLOGICALS 21(2): 103-4.
48. Rademacher, T. W., S. W. Homans, et al. (1986), Immunoglobulin Gas a glycoprotein, BIOCHEM SOC SYMP 51: 131-48.
49. Rademacher, T. W., R. B. Parekh, et al. (1988), Glycobiology, ANNUAL REV. BIOCHEM.57: 785-838.
50. Rademacher, T. W., R. B. Parekh, et al. (1988), The role of IgG glycoforms in the pathogenesis of rheumatoid arthritis, SPRINGER SEMIN IMMUNOPATHOL 10(2-3): 231-49.
51. Rudd, P. M., R. J. Leatherbarrow, et al. (1991), Diversification of the IgG molecule by oligosaccharides, MOL. IMMUNOL. 28(12): 1369-78.
52. REMINGTON'S PHARMACEUTICAL SCIENCES (16th ed., Osol, A., editor., Mack, Easton Press. (1980)).
53. Sacchi, S., M. Federico, et al. (2001), Treatment of B-cell non-Hodgkin's lymphoma with anti CD 20 monoclonal antibody Rituximab, CRIT. REV. ONCOL. HEMATOL. 37(1): 13-25.
54. Sambrook et al. (1989), MOLECULAR CLONING--A LABORATORY MANUAL, Cold Spring Harbor Laboratory Press, (2nd Edition).
55. Shen, E. S., G. M. Cooke, et al. (1995), Improved expression cloning using reporter genes and Epstein-Barr virus ori-containing vectors, GENE 156(2): 235-9.
56. Simmons, L. C., D. Reilly, et al. (2002), Expression of full-length immunoglobulins in Escherichia coli: rapid and efficient production of aglycosylated antibodies, J. IMMUNOL. METHODS 263(1-2): 133-47.
57. Shuford, W.W., et al. (1997), 4-1BB costimulatory signals preferentially induce CD8+ T cell proliferation and lead to the amplification in vivo of cytotoxic T cell responses, J. EXP. MED. 186:47.
58. Stanley, P. (1984), Glycosylation mutants of animal cells, ANNUAL REV. GENET. 18: 525-52.
59. Strome S.E. et al. (2002), Strategies for antigen loading of dendritic cells to enhance the antitumor immune response, CANCER RESEARCH 62:1884-9.
60. Sumar, N., K. B. Bodman, et al. (1990), Analysis of glycosylation changes in IgG using lectins, J. IMMUNOL. METHODS 131(1): 127-36.
61. Sutton, B. J. and D. C. Phillips (1983), The three-dimensional structure of the carbohydrate within the Fc fragment of immunoglobulin G, BIOCHEM SOC TRANS 11(Pt 2): 130-2.
62. Sun, Y. et al. (2002), Costimulatory molecule-targeted antibody therapy of a spontaneous autoimmune disease, NAT MED. 8(12), 1405-13.
63. Sun, Y. et al. (2002), Administration of agonistic aiiti-4-1BB monoclonal antibody leads to the amelioration of experimental autoimmune encephalomyelitis, J. IMMUNOL. 168(3), 1457-65.
64. Tamada K., et al. (2002), Cutting Edge: Selective impairment of CD8+ T cell function in mice lacking the tumor necrosis factor superfamily member LIGHT. J. IMMUNOL. 168:4832-5.
65. Tamada K. et al. (2000), Modulation of T cell-mediated immunity in tumor and graft versus host disease models through LIGHT costimulatory pathway, Nature Med. 6:283-9, 2000.
66. Tamada K. et al., LIGHT, a TNF-like molecule, costimulates T cell proliferation and is required for dendritic cell-mediated allogeneic T cell response, J.IMMUNOL. 164:4105-10.
67. Tandai, M., T. Endo, et al. (1991), Structural study of the sugar moieties of monoclonal antibodies secreted by human-mouse hybridoma, ARCH. BIOCHEM. BIOPHYS.291(2): 339-48.
68. Tao, M. H. and S. L. Morrison (1989), Studies of aglycosylated chimeric mouse-human IgG. Role of carbohydrate in the structure and effector functions mediated by the human IgG constant region, J. Immunol. 143(8): 2595-601.
69. Tao, M. H., R. I. Smith, et al. (1993), Structural features of human immunoglobulin G that determine isotype-specific differences in complement activation, J. EXP. MED. 178(2): 661-7.
70. Thornburg, R. W., J. F. Day, et al. (1980), Carbohydrate-mediated clearance of immune complexes from the circulation. A role for galactose residues in the hepatic uptake of IgG-antigen complexes, J. BIOL. CHEM. 255(14): 6820-5.
71. Tsuchiya, N., T. Endo, et al. (1989), Effects of galactase depletion from oligosaccharide chains on immunological activities of human IgG, J. RHEUMATOL. 16(3): 285-90.
72. Walker, M. R., J. Lund, et al. (1989), Aglycosylation of human IgG1 and IgG3 monoclonal antibodies can eliminate recognition by human cells expressing Fc gamma RI and/or Fc gamma RII receptors, BIOCHEM J. 259(2): 347-53.
73. Wang S., et al. (2002), Ligand binding sites of inducible costimulator and high avidity mutants with improved function, J. EXP. MED. 195:1033-41.
74. White, K. D., M. B. Frank, et al. (1996), Effect of immunoglobulin variable region structure on C3b and C4b deposition, MOL. IMMUNOL. 33(9): 759-68.
75. Wilcox R.A., et al. (2002), Provision of antigen and CD137 signaling breaks immunological ignorance, promoting regression of poorly immunogenic tumors, J. CLIN. INVEST. 109:651-9.
76. Wilcox R.A. et al. (2002), Expression of functional CD137 receptor by dendritic cells, J. IMMUNOL. 168:4262-7.
77. Wilcox R.A. et al. (2002), Signaling through NK cell-associated CD137 promotes both helper function for CD8+ cytolytic T lymphocytes and responsiveness to interleukin-2, J. IMMUNOL. 169:4230-6.
78. Wilmut I, et al. (Oct 10, 2002), Somatic Cell Nuclear Transfer, NATURE 419(6907):583-6.
79. Wilmut I, et al. (Feb 27, 1997), Viable Offspring Derived From Fetal and Adult Mammalian Cells, NATURE 385(6619):810-3.
80. Wilson et al. (2002), Provision of antigen and CD137 signaling breaks , immunological ignorance, promoting regression of poorly immunogenic tumors, J. CLIN. INVEST. 109:651-659.
81. Wright, A. and S. L. Morrison (1994), Effect of altered CH2-associated carbohydrate structure on the functional properties and in vivo fate of chimeric mouse-human immunoglobulin G1, J. EXP. MED. 180(3): 1087-96.
82. Zettlmeissl G, et al. (1991), Influence Of Glycosylation On The Functional Properties Of Human Therapeutic Plasma Proteins, GBF MONOGR. SER. 15:259.
83. Zhu G., et al. (2001), Progressive depletion of peripheral B lymphocytes in 4-1BB (CD137) ligand/I-Eβtransgenic mice, J. IMMUNOL. 167:2671-6.
84. Zou X, et al. (2002), Generation of Cloned Goats (Capra Hircus) From Transfected Foetal Fibroblast Cells, The Effect of Donor Cell Cycle, MOL REPROD DEV.; 61:164-172.

### Patents Incorporated by Reference

### Chen et al., 20050013811

Further aspects of the invention are described in the following clauses:
A. A recombinant anti-CD 137 antibody encoded by a transgene DNA construct in a first bioreactor organism comprising a polypeptide domain which is bioactive in a target animal when produced by said first bioreactor organism.
B. The recombinant antibody protein of clause A, wherein said transgenic antibody is the product of a contiguous coding sequence of DNA.
C. The transgenic antibody of clause B, wherein said antibody is glycosylated.
D. The transgenic antibody of clause B, wherein said antibody is aglycosylated.
E. The transgenic antibody of clause D wherein said recombinant antibody is used as a therapeutic agent in connection with bone marrow transplantation.
F. The transgenic antibody of clause C wherein said recombinant antibody is used as a therapeutic agent in connection with cancer immunotherapy or autoimmune disorders or cancerous lesions.
G. A method of treating a disease condition comprising administering to a mammal in need of such treatment a therapeutically effective amount of a compound of clause A or a prodrug thereof or a pharmaceutically acceptable salt of said compound or of said prodrug.
H. A method as recited in clause A wherein the amount of the Formula I compound is about 0.01 mg/kg/day to about 50 mg/kg/day.
I. A recombinant protein as recited in clause A wherein the therapeutic target animal is a human.
J. The method of clause A wherein said DNA construct encoding a desired transgenic protein is actuated by at least one beta-casein promoter.
K. The recombinant antibody protein of clause A, wherein said bioreactor organism is a transgenic ungulate.
L. A recombinant transgenic antibody produced by a method comprising:
   (a) expressing the transgenic antibody of clause A by a mammary epithelial cell; and
   (b) recovering the antibody.
M. The recombinant protein of clause A, wherein said recombinant antibody protein is expressed by a eukaryotic cell.
N. The transgenic protein of clause A, wherein said recombinant antibody protein is expressed by a eukaryotic cell in in vitro cell culture conditions.
O. The transgenic protein of clause N, wherein said recombinant antibody is expressed by an animal cell.
P. The transgenic protein of clause A, wherein said recombinant antibody is expressed by a prokaryote.
Q. A method for the production of transgenic animals capable of producing a transgenic antibody of interest comprising: transfecting a non-human mammalian cell-line with a transgene DNA construct encoding a desired transgenic antibody; selecting a cell line(s) in which said transgene DNA construct has been inserted into the genome of that cell or cell-line; and performing a first nuclear transfer procedure to generate a first transgenic animal heterozygous for the desired gene said animal being capable of expressing said transgenic antibody of interest in its milk.
R. The resultant milk derived from the offspring of the methods of clause Q.
S. The ungulate of clause K, wherein said bioreactor organism is selected from a group consisting of: goat, sheep, camel, cow, pig, rabbit, buffalo,horse, or llama.
T. The recombinant antibody of clause A further comprising a chimeric antibody.
U. The recombinant antibody of clause A further comprising a humanized antibody.
V. The recombinant antibody of clause A further comprising a fully human antibody.
W. A method for depleting double negative T-cells in a subject, said method comprising: (a) identifying a subject as being in need of depletion of double negative T cells; and (b) administering to said subject an effective amount of a recombinant agonist anti CD 137 antibody.
X. The method of clause W, wherein said subject is a human.
Y. The method of clause W, wherein said subject has or is at risk of having an autoimmune disease, a lymphoproliferative disease, or an allergy and wherein the recombinant antibody so administered is aglycosylated.
Z. The method of clause Y, wherein said autoimmune disease or said lymphoproliferative disease is selected from the group consisting of systemic lupus erythematosus, insulin-dependent diabetes mellitus, an inflammatory bowel disease, a celiac disease, an autoimmune thyroid disease, Sjogren's Syndrome, autoimmune gastritis, pernicious anemia, autoimmune hepatitis, cutaneous autoimmune diseases, autoimmune dilated cardiomyopathy, myocarditis, myasthenia gravis, vasculitis, autoimmune diseases of the muscle, autoimmune diseases of the testis, autoimmune diseases of the ovary, and autoimmune diseases of the eye.

AA. The method of clause Y, wherein said allergy is to pollen antigens, fungal antigens, insect antigens, bacterial antigens, mammalian antigens, or insect venom antigens.
AB. The recombinant antibody of clause F, further comprising wherein said cancerous conditions include: lymphoma, leukemia, mastocytoma, thymoma, melanoma, renal carcinoma, plasmacytoma, breast cancer, colon cancer and glioma.

## Claims

1. A transgenic anti-CD 137 antibody, wherein the antibody is glycosylated in the Fc region.

2. The transgenic antibody of claim 1, wherein the antibody is a chimeric antibody.

3. The transgenic antibody of claim 1, wherein the antibody is a humanized antibody, preferably a fully humanized antibody.

4. The transgenic antibody of claim 1, wherein the antibody is encoded by a contiguous coding sequence of DNA, preferably wherein the coding sequence of DNA is actuated by at least one beta-casein promoter.

5. The transgenic antibody of claim 1, wherein the transgenic antibody is produced in non-human mammalian epithelial cells.

6. The transgenic antibody of claim 1, wherein the transgenic antibody is produced in a transgenic animal and wherein the transgenic animal is selected from the group consisting of a mouse, a goat, a sheep, a camel, a cow, a pig, a buffalo, a horse, a swine, cattle, a hamster, a rabbit, a guinea pig, a rat, a non-human primate, and a llama.

7. The transgenic antibody of claim 1, wherein the transgenic antibody is produced in an ungulate; preferably wherein the ungulate is selected from the group consisting of a goat, a sheep, a camel, a cow, a pig, a buffalo, a horse and a llama; and most preferably wherein the ungulate is a goat.

8. The transgenic antibody of claim 1, wherein the transgenic antibody is produced in a mouse.

9. A method for producing the transgenic antibody of claim 1, the method comprising:
expressing a nucleic acid sequence encoding the transgenic antibody in a mammary epithelial cell; and,
recovering the antibody.

10. The transgenic antibody of claim 1, for use in the treatment of autoimmune disorders.

11. The transgenic antibody of claim 1, for use in connection with bone marrow transplantation.

12. The transgenic antibody of claim 1, for use in the treatment of cancer, preferably wherein the cancer is lymphoma, leukemia, mastocytoma, thymoma, melanoma, renal carcinoma, plasmacytoma, breast cancer, colon cancer, a solid tumor, ovarian carcinoma, squamous cell carcinoma or glioma.

13. The transgenic antibody of claim 1, for use in cancer immunotherapy.

14. A composition comprising the transgenic antibody of claim 1, and a pharmaceutically acceptable carrier.

15. A composition comprising milk and the transgenic antibody of claim 1.
